(19) Europäisches Patentamt / European Patent Office / Office européen des brevets

(11) **EP 4 310 174 A1**

(12) **EUROPEAN PATENT APPLICATION**

(43) Date of publication:
24.01.2024 Bulletin 2024/04

(21) Application number: 22185402.9

(22) Date of filing: 18.07.2022

(51) International Patent Classification (IPC):
*C12N 1/00* (2006.01)

(52) Cooperative Patent Classification (CPC):
**C12N 15/52; C07K 14/195; C07K 14/245;
C12N 1/20; C12P 7/00;** C12R 2001/01;
C12R 2001/19

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **ETH Zurich
8092 Zurich (CH)**

(72) Inventors:
• **Vorholt-Zambelli, Julia Anne
8044 Zürich (CH)**
• **Keller, Philipp
6300 Zug (CH)**
• **Reiter, Michael Alexander
8057 Zürich (CH)**

(74) Representative: **P&TS SA (AG, Ltd.)
Avenue J.-J. Rousseau 4
P.O. Box 2848
2001 Neuchâtel (CH)**

Remarks:
The complete document including Reference Table(s) and the Sequence Listing(s) can be downloaded from the EPO website

(54) **RECOMBINANT METHYLOTROPHIC MICROORGANISM**

(57) This invention concerns a recombinant non-naturally occurring methylotrophic microorganism, which grows on reduced one-carbon (C1) compounds as sole carbon source. The recombinant microorganism expresses or over-expresses a polypeptide having methanol dehydrogenase activity, a polypeptide having 3-hexulose-6-phosphate activity, and polypeptide having a 6-phospho 3-hexuloisomerase activity, and does not comprise, and/or comprises deletions or reductions of expression of, or eliminations or reductions of activity of a polypeptide having triose-phosphate-isomerase activity, and of a polypeptide having glutathione-dependent formaldehyde dehydrogenase activity.

Fig. 2

EP 4 310 174 A1

## Description

## Technical domain

The present disclosure concerns a metabolically modified microorganism. The present disclosure also concerns a method of converting reduced one-carbon compounds into carbon metabolites.

## Related art

Biotechnology is a key sector of the 21st century and is expected to expand massively in the coming decades. However, the currently applied biotransformation processes conflict with the production of human and animal foodstuffs, as the production of the used raw materials predominantly relies on the exploitation of agricultural land.

Therefore, alternative non-food and non-feed sources are required to replace sugar as the main substrate. Reduced one-carbon compounds, also called C1 compounds, are abundant in nature and as by-products of industrial processes. Their high availability, low production cost and high energy density makes them ideal source materials to produce feedstock or commodity chemicals. However, most biotechnologically relevant microorganisms lack functional assimilation pathways to convert reduced C1 compounds into metabolites and biomass.

Methanol in particular holds promise as an alternative substrate to replace sugars in the biotechnology industry. At ambient temperatures methanol is liquid with high energy storage capacity. It can be produced from carbon dioxide ($CO_2$) or from methane and its use does not compete with food and animal feed production.

Moreover, the bioconversion of methanol also contributes to the reduction of greenhouse gases, as it can assist in reducing the amount of methane and $CO_2$ in the atmosphere, paving the way for sustainable biotechnological processes.

Organisms capable of using methanol to grow and to build their biomass, referred to as methylotrophs, are abundant in nature, however, their biotechnological application is limited due to the lack of advanced genetic tools for these organisms.

An alternative to relying on natural methylotrophs for the assimilation of reduced C1 compounds is to enable strains more commonly used in biotechnological processes, such as *Escherichia coli* (*E. coli*), to metabolize methanol. The generation of genetically engineered methylotrophs has attracted considerable attention in the past few years.

Several attempts describe this adaptation of bacterial strains, which are not naturally capable of growing on methanol, into at least partially methylotrophic strains or methanol-dependent strains.

Kim S. et al., Nat. Chem. Biol. (2020), DOI:10.1038/s41589-020-0473-5 describes a genetically altered *E. coli* strain, which grows on formate and methanol. The authors combined rational engineering with adaptive laboratory evolution to develop a strain capable of growth on formate via the reductive glycine pathway. Additional heterologous expression of a methanol dehydrogenase rendered this strain capable of growing on methanol, albeit with a high doubling time of about 54 hours.

WO2018148703 discloses a method for increasing production of a metabolite by a non-naturally occurring methylotroph when grown on a medium comprising methanol. However, the engineered strains described in this document require additional carbon sources, which are not C1 compounds, such as glucose or ribose, to grow on methanol. It is therefore evident, that only a small portion of the bacterial biomass and metabolites of this strain are derived from methanol.

WO2022015796 describes metabolically modified microorganisms that can grow on an organic C1 carbon source. However, it is doubted that the modified bacterial strain disclosed in this document is indeed capable of growing on methanol as a sole carbon source, since it the strain was evolved in a medium containing 50 mM 4-morpholinepropanesulfonic acid and 4 mM tricine, which may have served as additional carbon sources. The modification of the disclosed strain comprises primarily copy number variations of large parts of the genome. The copy number variations in the evolved strain resulted in genomic instability, i.e. after cultivation of this strain in rich medium lacking methanol the strain exhibited slower growth on minimal medium containing methanol.

In Keller, P. et al., 2020, Nat. Commun. 11, 5403, https://doi.org/10.1038/s41467-020-19235-5, a recombinant methanol-dependent *Escherichia coli* strain comprising specific gene deletions and expressing a set of heterologous polypeptides, is described. The strain requires methanol for growth but needs to be provided with further multi-carbon substrates for growth from which the majority of the biomass is formed.

The studies mentioned here were at least partially successful in generating non-naturally occurring methanol-dependent microorganisms, which are able to assimilate methanol.

However, each of the strains described in the state of the art is fraught with considerable drawbacks, such as slow growth, assimilation of only a small portion of reduced C1 compounds into biomass, or genomic instability, which impair the effectiveness of conversion of reduced C1 compounds into biomass.

It is an object of this invention to overcome the shortcomings and limitations of the state of the art.

**Short disclosure of the invention**

It is an aim of the present invention to render the conversion of reduced C1-compounds, such as methanol, into biomass more efficient.

It is another aim of the present invention to provide a genetically modified methylotrophic microorganism, which is capable of producing biomass and metabolites from reduced C1 compound(s), in particular methanol. Preferably the strain should be fully methylotrophic, i.e. capable of producing all its carbon metabolites exclusively from C1 substrates. Preferably, the stain should be suitable for industrial use.

It is another aim of this invention to provide a genetically modified methylotrophic microorganism with good genomic stability. The strain should be more stable than other non-naturally occurring genetically modified methylotrophs known in the art.

It is a further aim of this invention to provide an alternative genetically engineered methylotrophic microorganism.

According to the invention, one or more of these aims are attained by the object of the attached claims, and especially by the independent claims.

In particular, one or more of these aims are attained by a recombinant methylotrophic microorganism, which grows on a one-carbon (C1) compounds as its sole source of carbon, wherein at least 50% of the carbon source consists of one or more reduced C1 carbon compounds. The recombinant methylotrophic microorganism does not require any multi-carbon sources for growth.

The recombinant methylotroph expresses or over-expresses a polypeptide having methanol dehydrogenase activity, such as Mdh, a polypeptide having 3-hexulose-6-phosphate activity, such as Phi, and polypeptide having a 6-phospho 3-hexuloisomerase activity, such as Hps.

The recombinant methylotroph does either (i) not comprise, (ii) has deletions in genes expressing, (iii) has a reduction in expression of, and/or (iv) has an elimination or a reduction of enzymatic activity of both of the following polypeptides:

- a polypeptide having triose-phosphate-isomerase activity, such as TpiA, and

- a polypeptide having glutathione-dependent formaldehyde dehydrogenase activity, such as FrmA.

Each of the polypeptides having a methanol dehydrogenase activity, a polypeptide having 3-hexulose-6-phosphate activity, or a polypeptide having a 6-phospho 3-hexuloisomerase activity may be expressed or over-expressed heterologously. It is also possible that one or more of these polypeptides are expressed or overexpressed homologously.

Reduced C1-compounds consist of methanol or formaldehyde, as well as substrates which are converted into methanol or formaldehyde for biological assimilation, such as methane, dichloroethane, as well as methylated compounds, where the non-methyl moiety is not metabolized, such as sarcosine.

Heterologous expression refers to the expression of a gene in an organism which does not naturally comprise this gene. Homologous expression refers to expression or over-expression of a gene in an organism naturally comprising the gene.

When grown on methanol, the biomass and metabolites produced by the recombinant methylotroph should preferably comprise at least 70%, at least 75%, at least 80%, preferably 90% $\pm$ 5% of carbon retrieved from reduced C1 compounds, such as methanol. Preferably, the remainder of the carbon converted into biomass and metabolites is derived from another C1 source, for example from $CO_2$.

The recombinant methylotroph of this invention is capable of growing on carbon sources consisting of C1 compounds. Different from the recombinant methylotrophs described in the prior art, the strain provided by this invention is capable of producing its entire biomass and metabolites solely from C1 compounds, such as methanol and $CO_2$. When methanol is the only compound provided as a carbon source in the growth medium, the strain is preferably able to assimilate $CO_2$ from atmosphere for the production of its metabolites and biomass. No multi-carbon source, i.e. a carbon source with more than one carbon atom, is required for growth.

The recombinant methylotrophic strain expresses or overexpresses, at least in part heterologously, all enzymes required for a complete ribulose monophosphate (RuMP) cycle, a pathway used for the assimilation of reduced one-carbon compounds in natural methylotrophs. Such enzymes include a polypeptide having methanol dehydrogenase activity, such as Mdh, a polypeptide having 3-hexulose 6-phosphate synthase, such as Hps, a polypeptide having 6-phospho 3-hexuloisomerase activity, such as Phi, a polypeptide having glucose-6-phosphate isomerase activity, such as Pgi, a polypeptide having transketolase activity, such as TktA, a polypeptide having ribulose-phosphate 3-epimerase, such as Rpe, a polypeptide having ribose-5-phosphate isomerase activity, such as RpiA, a polypeptide having transaldolase activity, such as TalA, a polypeptide having NADP+-dependent glucose-6-phosphate dehydrogenase, such as Zwf, a polypeptide having 2-keto-3-deoxygluconate 6-phosphate/2-keto-4-hydroxyglutarate aldolase activity, such as Eda, and a polypeptide having a phosphogluconate dehydratase, such as Edd.

In *E. coli,* the heterologous expression of an enzyme having a methanol dehydrogenase activity, such as Mdh, an enzyme having a 3-hexulose 6-phosphate synthase, such as Hps, and an enzyme having a 6-phospho 3-hexuloisomerase activity, such as Phi, enable the influx of C1 compounds into the RuMP cycle and complete the RuMP cycle. When expressing these enzymes, a recombinant *E. coli* strain can assimilate C1 substrates to some extent.

However, in *E. coli* and in other non-naturally occurring methylotrophs, the heterologous expression of an enzyme having a methanol dehydrogenase activity, such as Mdh, an enzyme having a 3-hexulose 6-phosphate synthase, such as Hps, and an enzyme having a 6-phospho 3-hexuloisomerase activity, such as Phi, is insufficient to allow growth of *E. coli* wild type strains, which are naturally non-methylotrophic, on methanol. This can be attributed to insufficient flux of formaldehyde flux into the RuMP cycle and the imbalanced efflux of RuMP cycle intermediates.

To overcome this problem, further mutations need to be introduced. Suitable mutations are mutations resulting in disruptions in expression or activity of a polypeptide having glutathione-dependent formaldehyde dehydrogenase, such as FrmA, , and of a polypeptide having triose phosphate isomerase activity, such as TpiA, as mentioned above. The mutations are preferably deletions of the gene encoding glutathione-dependent formaldehyde dehydrogenase, *frmA,* and the gene encoding the native triose phosphate isomerase, *tpiA,* of the recombinant microorganism.

A suitable mutation in or deletion of the native *tpiA* gene interrupts gluconeogenesis and abolishes growth on multi-carbon compounds including pyruvate, succinate, and/or acetate in the absence of methanol. The strain thereby enables methanol-dependent growth in presence of methanol and the multicarbon source pyruvate.

A suitable mutation in or deletion of the native *frmA* gene blocks the formaldehyde detoxification pathway, ensuring high levels of formaldehyde, the one-carbon compound that enters the RuMP cycle.

To further enhance the efficiency of the recombinant methylotroph described above, and/or to achieve a stable assimilation of methanol the strain preferably comprises further mutations. Such mutations may alter the expression or activity of polypeptides coordinating, be it as enzymes or as regulators, the carbon flux through the autocatalytic RuMP cycle.

The efficiency of C1 assimilation can be increased by additionally modifying the recombinant microorganism, to express or over-express one or more polypeptides selected from the group consisting of Mdh, Hps, Phi, TktA, TktB, Rpe, RpiA, RpiB, TalA, TalB, Edd, and Eda,or any combination thereof. Preferably these polypeptides are expressed from their corresponding native genes. It is however also possible to transform the recombinant organism with one or more of the genes for heterologous expression of the encoded polypeptides.

The recombinant methylotroph may for example express or over-express a polypeptide having TktA activity and a polypeptide having RpiB activity, or a polypeptide having TktA activity and a polypeptide having Edd activity and/or polypeptide having Eda activity, or a polypeptide having RpiB and a polypeptide having Edd activity and/or polypeptide having Eda activity.

The recombinant methylotroph may comprise one or more modifications in one or more native genes increasing the enzymatic activity of the polypeptide encoded by the mutated gene. The recombinant strain may for example comprise modifications increasing activity of polypeptides encoded by one or more native genes selected from the group consisting of *rpe, rpiB, tktA,* and *tktB,* or any combination thereof.

The recombinant methylotroph may comprise one or more modifications in one or more native genes decreasing or eliminating the enzymatic activity of the polypeptide encoded by the mutated gene. This modification may for example be a point mutation, a mutation leading to a truncated polypeptide, or a deletion of the gene or portions thereof. The recombinant strain may for example comprise modifications decreasing or eliminating activity of polypeptides encoded by one or more native genes selected from the group consisting of *pgi, gnd, gntR, pfkA, pgk, aroF, aroH, prs, purF, aceA, acnA, fumC, sdhB, sucA, sucC,* and *icd,* or any combination thereof.

The recombinant methylotroph may comprise a modification in the *gntR* gene decreasing or eliminating the activity of DNA-binding transcriptional repressor GntR. Preferably, the modification prevents the dimerization of the repressor. The modification may, for example, be a mutation resulting in the truncation of the GntR repressor.

The efficiency of C1 assimilation in the recombinant methylotroph described above can be enhanced by introducing further modifications to reduce or eliminate the expression of one or more polypeptides from native genes selected from the group consisting of *gntR, tktB, pgi, pkg, pgl, gnd, aroH, aroF, pfkA, prs, purF, a gene encoding an enzyme of the* tricarboxylic acid (TCA) cycle, including *aceA, acnA, fumC, sdhB, sucA, sucC, icd, aceE, aceF, sdhA, sucC, and mqo, or* any combination thereof.

The recombinant methylotroph may for example comprise a reduction in expression, or a deletion of the gene encoding the Gnd polypeptide and in the gene encoding the AroH polypeptide. Alternatively, it may for example comprise a reduction in expression of the gene encoding the AcnA polypeptide and the gene encoding the Pgi polypeptide. It may comprise a reduction in the expression of the gene encoding the AcnA polypeptide and the gene encoding the Pgl polypeptide. It may comprise a reduction in the expression of the gene encoding the Pgi polypeptide and the gene encoding the Pgl polypeptide. It may comprise a reduction in the expression of the gene encoding the AcnA polypeptide, the gene encoding the Pgi polypeptide, and the gene the gene encoding the Pgl polypeptide.

The recombinant methylotroph may express or over-express an Mdh polypeptide having at least 70%, 80%, 90%, 95%, 98% or 99% sequence identity to SEQ ID NO: 1, wherein the polypeptide preferably comprises at least an H165N mutation.

It was established as part of this invention, that a Mdh polypeptide comprising this mutation has an improved methanol dehydrogenase activity compared to the parental polypeptide. By introducing an H165N mutation in a Mdh polypeptide, the methanol dehydrogenase activity compared to the parental Mdh polypeptide can be increased by at least 1.5-fold, or at least 2-fold, or at least 2.5-fold.

Preferably, the non-naturally occurring recombinant methylotroph can tolerate methanol concentration of 1 M. The recombinant methylotroph is preferably able of growing in medium supplemented with up to 1 M M methanol, for example between 100 mM and 2 M methanol, or any value between any of the two foregoing values.

Preferably the recombinant methylotroph is grown under atmospheric conditions in or on a medium provided with methanol as sole carbon source.

In one aspect of this invention the non-naturally occurring recombinant methylotroph is capable of reaching an optical density at 600 nm ($OD_{600}$) of 2 or more, or of 2.5 or more when grown under atmospheric conditions in a medium comprising only 500 mM methanol as carbon source.

In one aspect of this invention the non-naturally occurring recombinant methylotroph is capable of growing on methanol at a doubling time of 10 hours or less, preferably of 8 hours or less.

The recombinant non-naturally occurring methylotroph may be obtained by genetically modifying a non-naturally occurring methylotrophic microorganism selected from a group consisting of *Escherichia,* in particular *Escherichia coli, Bacillus,* in particular *Bacillus subtilis, Clostridium, Enterobacter, Klebsiella, Mannheimia, Pseudomonas,* in particular, *Pseudomonas putida, Acinetobacter, Shewanella, Paracoccus, Ralstonia,* for example *R. eutropha, Geobacter, Zymomonas, Acetobacter, Geobacillus, Lactococcus, Streptococcus, Lactobacillus, Corynebacterium,* in particular *Corynebacterium glutamicum, Streptomyces, Proprionibacterium, Synechocystis, Synechococcus, Cyanobacteria, Chlorobi, Deinococcus* and *Saccharomyces sp.*

The non-naturally occurring recombinant methylotroph of this invention may be a synthetic methylotrophic microorganism.

The recombinant non-naturally occurring methylotroph may be an *Escherichia coli* strain. The recombinant non-naturally occurring methylotroph may be the strain *E. coli* strain designated MEcoli_ref_1 having the Culture Collection of Switzerland (CCOS) accession number CCOS 2032. The recombinant non-naturally occurring methylotroph may be an *E. coli* strain having a doubling time and/or product profile which is similar to or substantially the same as the doubling time and/or the product profile of *E. coli* CCOS deposit having accession number CCOS 2032, when grown on methanol.

The invention also concerns a method for producing or for increasing production of a metabolite, comprising the steps of growing a recombinant non-naturally methylotrophic microorganism described herein on a C1 substrate and obtaining a metabolite.

The metabolite may for example be obtained from the growth supernatant of the cultured recombinant methylotroph. In addition or alternatively, the metabolite may be obtained from the biomass and/or from lysed recombinant methylotroph.

The one or more metabolites obtainable by this method will depend on the ancestral microorganism which was modified to generate the recombinant non-naturally occurring methylotroph of this invention. When modified according to this invention, the metabolites produced by these strains are generated by providing C1 compounds as principal or sole source for carbon.

The metabolites may for example be selected from a group consisting of 2-carbon compounds, 3-carbon compounds, 4-carbon compounds, higher carboxylic acids, alcohols of higher carboxylic acids, polyhydroxyalkanoates, and speciality chemicals, aromatic compounds, vitamins and their derivatives or precursors, and carotenoides.

The metabolites may for example be selected from a group consisting of formate, lactate, amino acids or sugars, such as deoxyribose.,

With respect to what is known in the art, the invention provides the advantage that the recombinant non-naturally methylotrophic microorganism is fully methylotroph, i.e. it uses C1 substrates as its sole source for carbon. It is therefore more efficient than the recombinant methanol-dependent and methylotrophic strains known in the art in converting C1 compounds, such as methanol, into biomass.

Moreover, a recombinant non-naturally methylotrophic microorganism disclosed according to this invention is also more efficient than previously described recombinant non-naturally methylotrophic microoorganisms in converting C1 compounds into metabolites, which may be industrially relevant metabolites. In addition, since the methylotrophy of the recombinant microorganism does not rely on gene copy number variations of the modified strain, which are notoriously instable, the recombinant methylotroph disclosed herein is also genetically more stable than previously described recombinant methylotrophic microorganisms.

Growth of the recombinant non-naturally methylotrophic microorganism disclosed according to this invention on methanol is therefore more robust than methanol-dependent growth of previously disclosed recombinant non-naturally methylotrophic strains.

## Short description of the drawings

Exemplar embodiments of the invention are disclosed in the description and illustrated by the drawings in which:

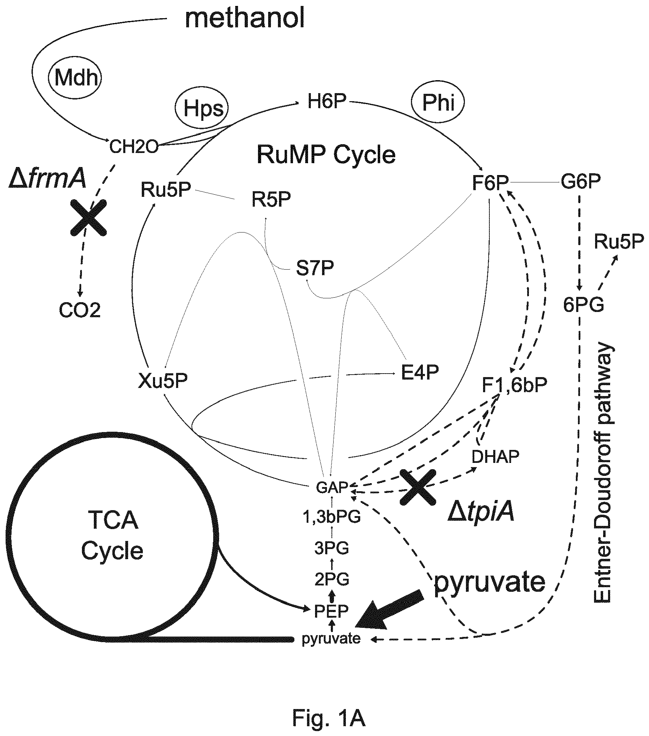

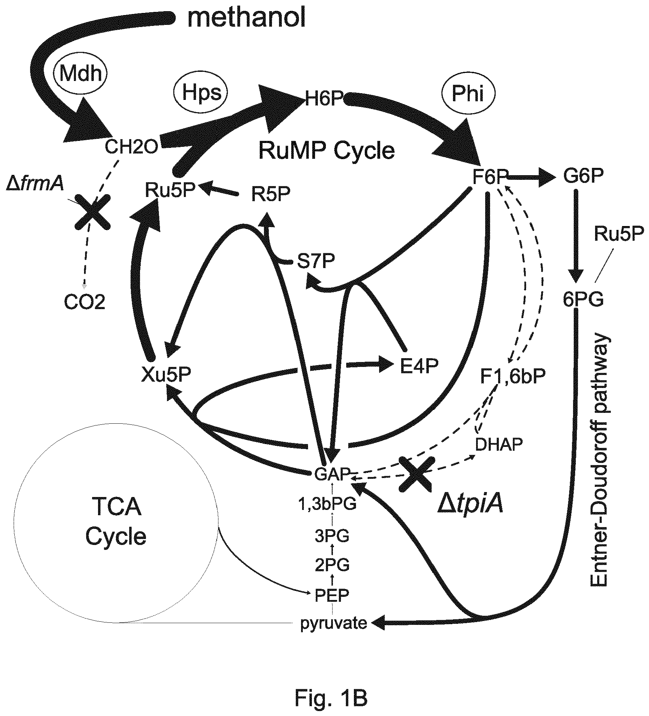

**Figures 1A** and **1B** are a graphic presentation of the RuMP cycle, illustrating flux variability analysis (FVA)-predicted differences of the flux distribution of the methanol-dependent *E. coli* strain *ΔfrmAΔtpiA* when grown on methanol and pyruvate (Figure 1A), and when grown on methanol alone (Figure 1B).

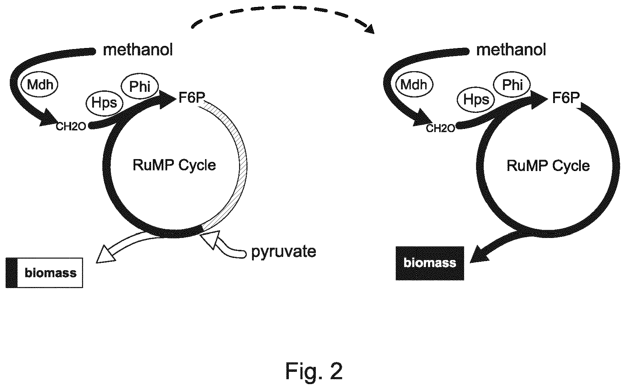

**Figure 2** represents schematically the adaptation of the methanol-dependent *E. coli* strain *ΔfrmAΔtpiA* during evolution; the diagram on the left shows fluxes before long-term laboratory evolution, the diagram on the right shows fluxes following successful long-term laboratory evolution; long-term laboratory evolution is indicated by a dashed arrow;

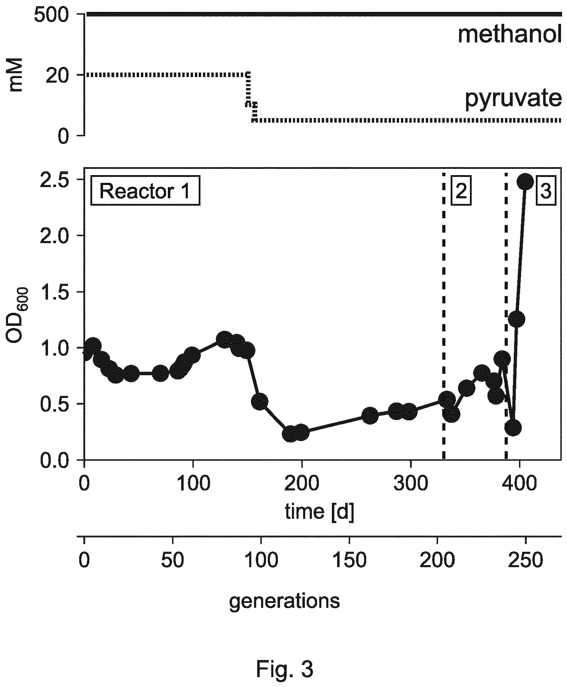

**Figure 3** depicts the culture density achieved by different generations of the evolving *E. coli* strain *ΔfrmAΔtpiA* grown on methanol and pyruvate in continuous chemostat culture. Once methylotrophic growth was achieved after about 250 generations of evolution, the optical density increased.

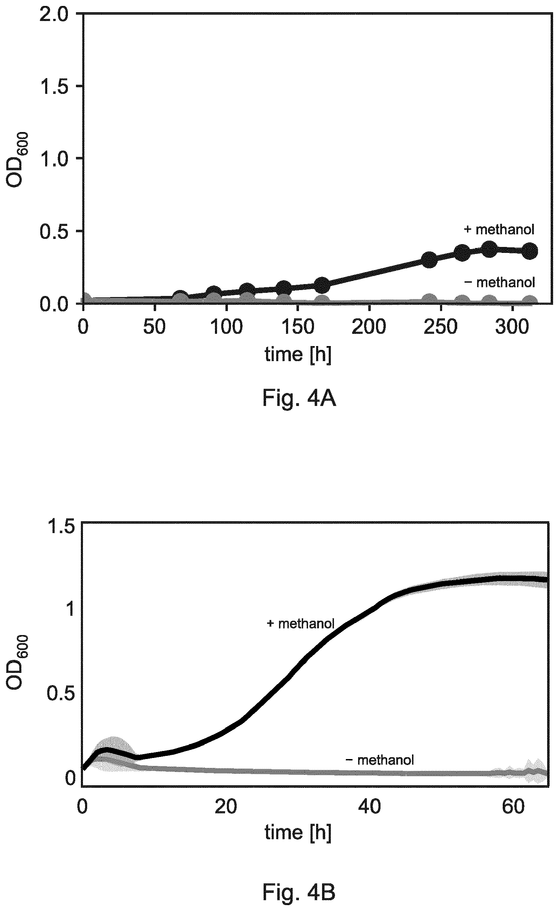

**Figures 4A and 4B** show growth curves of the evolving *E. coli* strain *ΔfrmAΔtpiA* in the absence of pyruvate, in minimal medium without methanol or supplemented with 500 mM methanol.

Figure 4A shows growth of the evolved population after 249 generations in the presence and absence of methanol.

Figure 4B shows a mean of 10 growth curves of the evolved strain MEcoli_ref_1 in the presence and absence of methanol.

**Figure 5A** show genetic and proteome changes of a recombinant methylotrophic *E. coli* strain MEcoli_ref_1; Proteomics data, indicating the fold change in expression, are indicated left to the corresponding gene.

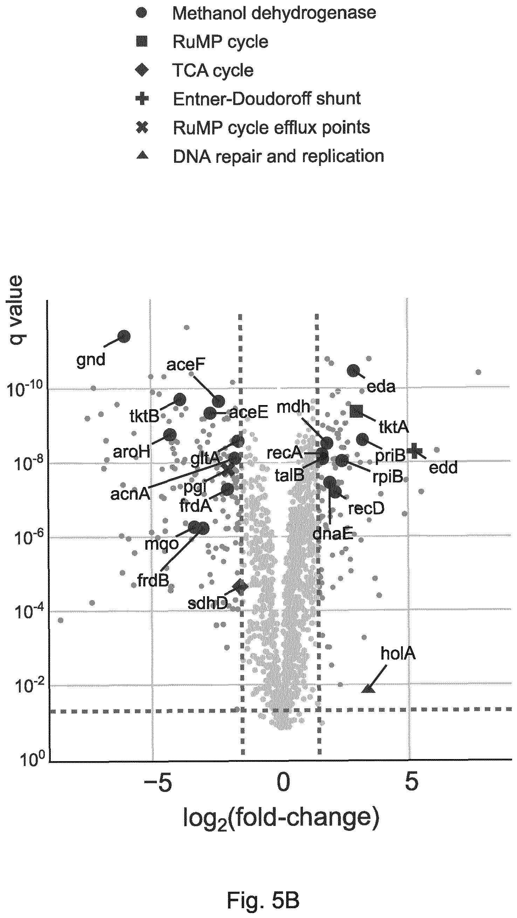

**Figure 5B** depicts a comparison of proteomes between the ancestral methanol-dependent *E. coli* strain *ΔfrmAΔtpiA* and the fully methylotrophic *E. coli* strain MEcoli_ref_1; the ancestral methanol-dependent *E. coli was* grown in minimal medium supplemented with 500 mM methanol and 20 mM pyruvate; the methylotrophic strain MEcoli_ref_1 was grown in minimal medium containing only 500mM methanol as carbon source; differentially expressed genes (log2(fold-change) ≥ 1.5) of important metabolic pathways and the enriched supergroup of DNA repair and replication are indicated with different symbols;

**Figures 6A to 6D** show methanol incorporation into protein-bound amino acids by the evolved methylotrophic *E. coli* strain in isotope distributions (Figures 6A and 6C) and in $^{13}C$ fractional distributions (Figures 6B and 6D);

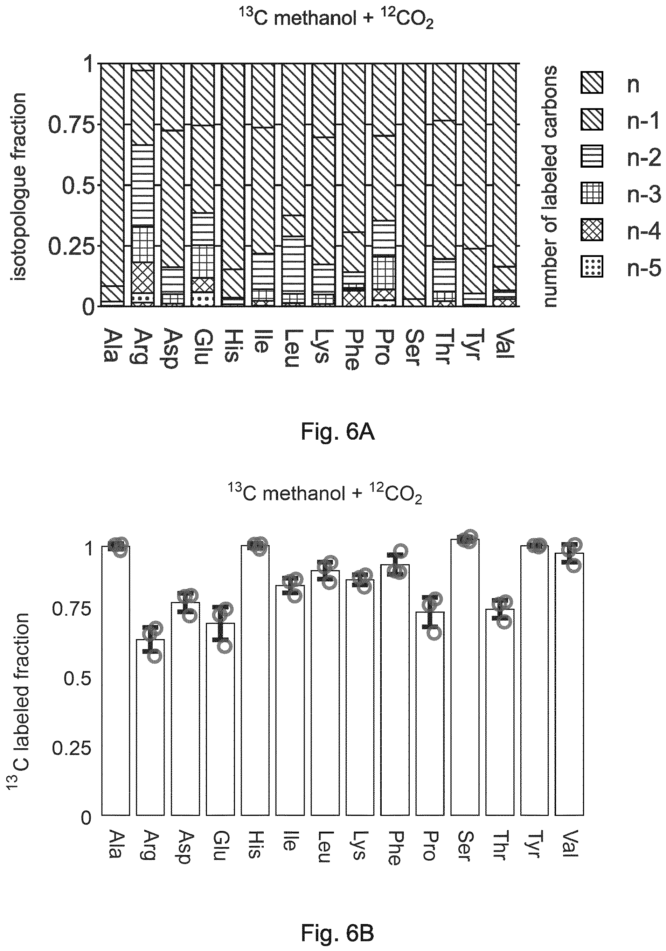

Figures 6A and 6B show MEcoli_ref_1 grown in minimal medium supplemented with 500 mM $^{13}C$ methanol either under ambient $CO_2$;

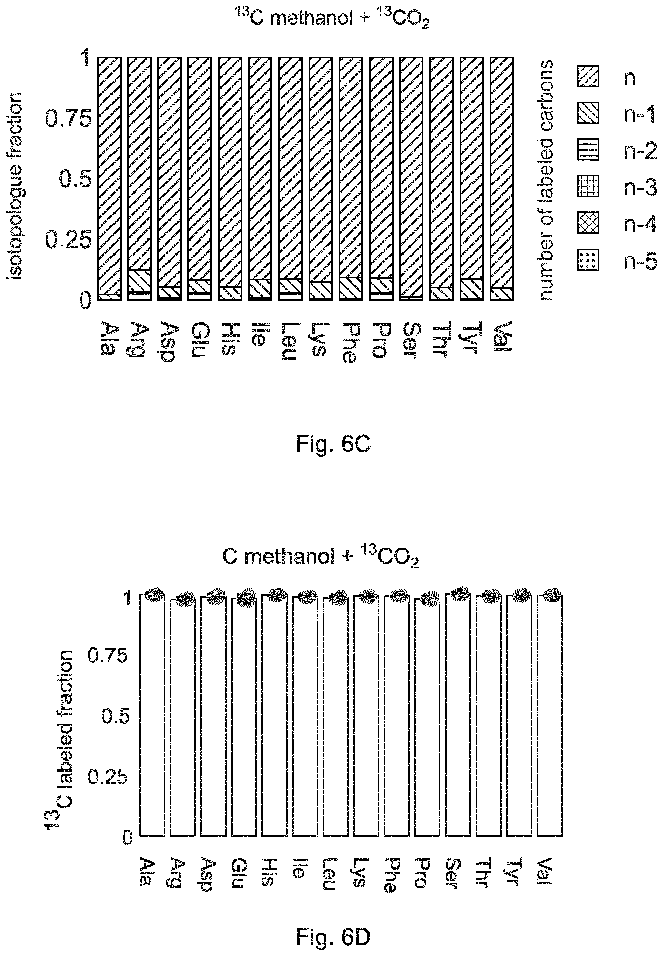

Figures 6C and 6D show MEcoli_ref_1 grown in minimal medium supplemented with 500 mM $^{13}C$ methanol under 5% (V/V) enriched $^{13}CO_2$ atmosphere.

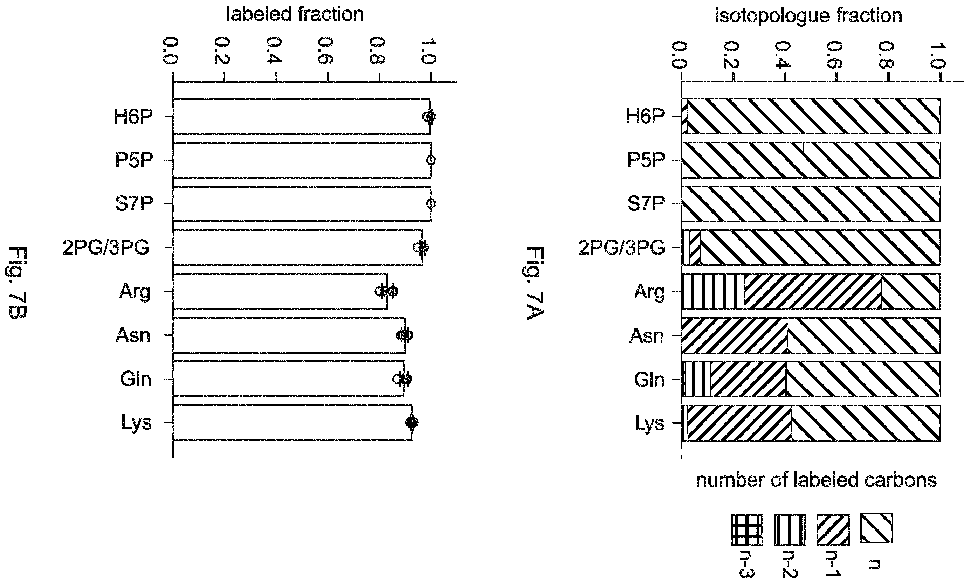

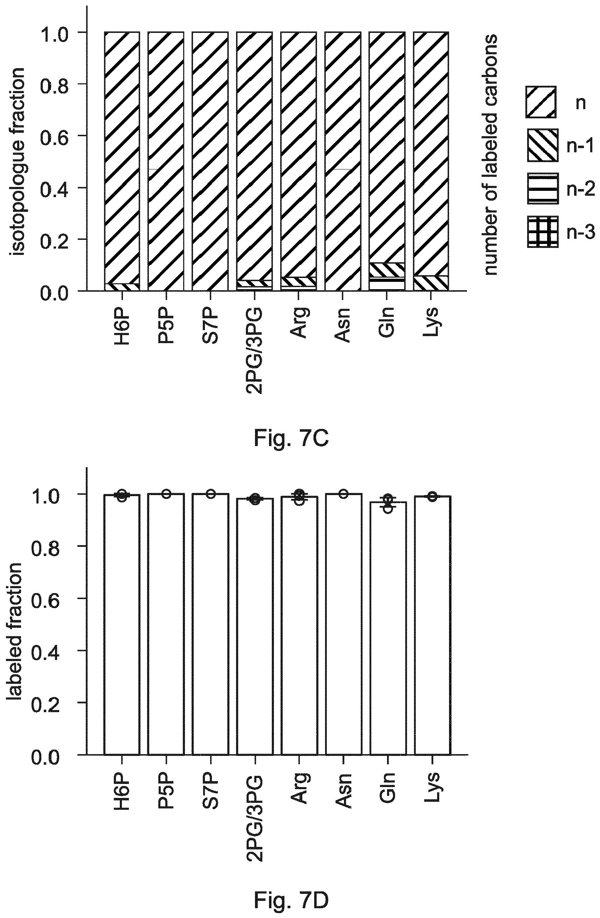

**Figures 7A to 7D** show methanol incorporation into protein-bound intermediates by the evolved methylotrophic *E. coli* strain in isotope distributions (Figures 7A and 7C) and in $^{13}C$ fractional distributions (Figures 7B and 7D);

Figures 7A and 7B show MEcoli_ref_1 grown in minimal medium supplemented with 500 mM $^{13}C$ methanol either under ambient $CO_2$;

Figures 7C and 7D show MEcoli_ref_1 grown in minimal medium supplemented with 500 mM $^{13}C$ methanol under 5% (V/V) enriched $^{13}CO_2$ atmosphere.

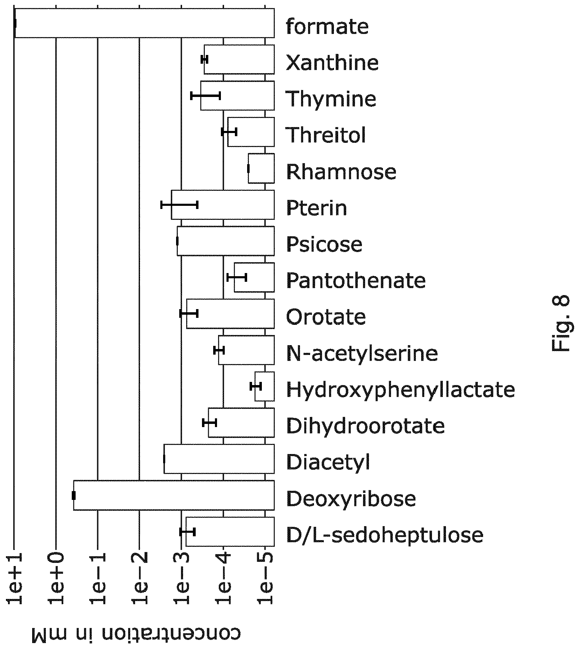

**Figure 8** shows a series of metabolites in the growth supernatant of the evolved methylotrophic *E. coli* strain;

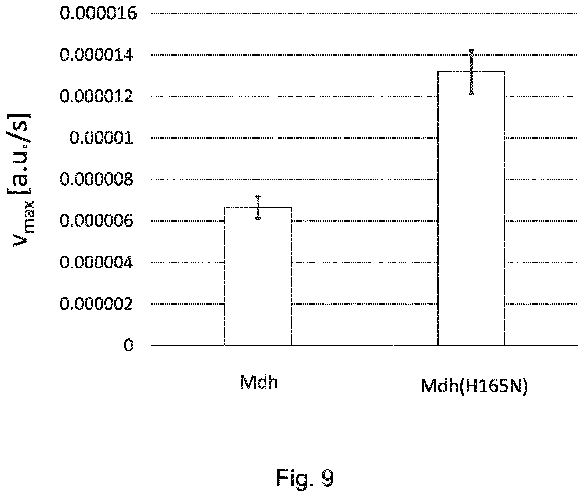

**Figure 9** shows a comparison of catalytic turnover between ancestral dehydrogenase Mdh and mutated methanol dehydrogenase Mdh(H165N);

**Examples of embodiments of the present invention**

A "recombinant microorganism" refers to a microorganism which has been genetically modified to express or over-express endogenous polynucleotides, or to express non-endogenous nucleotide sequences, such as those included in a plasmid, or which has a reduction in expression of an endogenous gene.

A "synthetic microorganism" refers to a recombinant microorganism in which a substantial portion of the genome or the entire genome has been genetically modified.

A" non-naturally occurring methylotrophic microorganism" refers to a microorganism derived from an ancestral microorganism which lacks the ability of methanol-dependent growth or of fully methylotrophic growth, but through recombinant engineering or recombinant engineering and laboratory evolution has been adapted to full methylotrophic growth.

The term "recombinant methylotroph" as used herein refers to a non-naturally occurring genetically modified methylotroph.

A "methylotrophic microorganism" is a microorganism which converts C1 compounds, in particular methanol, into biomass.

The term "methanol-dependent microorganism" refers to a microorganism which requires methanol as well as other multi-carbon substrates for growth. Even though this microorganism requires methanol for growth, it is unable to grow on C1 compounds as sole carbon source. "Methanol-dependent" therefore indicates the requirement for methanol and at least one multi-carbon compound for growth.

The term "fully methylotrophic microorganism" is a microorganism which uses exclusively reduced C1 compounds as carbon source. Preferably, the main C1 carbon source for a methylotroph is methanol. "Fully methylotroph" or "full methylotrophy" therefore indicate that only C1-compounds, in particular methanol, are required as carbon substrates for growth.

Organisms capable of using methanol as a growth substrate, referred to as methylotrophs, are abundant in nature; however, their biotechnological application is limited due to the lack of advanced genetic tools. An alternative to relying on natural methylotrophs is to enable an already established platform organism, such as *Escherichia coli,* to metabolize methanol. The generation of synthetic methylotrophs has attracted considerable attention in the past few years and has mainly focused on the introduction of the ribulose monophosphate (RuMP) cycle for carbon assimilation due to its superior efficiency compared to alternative carbon assimilation pathways.

In *E. coli,* only three genes encoding a methanol dehydrogenase (*mdh*), a 3-hexulose 6-phosphate synthase (*hps*), and a 6-phospho 3-hexuloisomerase (*phi*) are lacking for a complete RuMP cycle. Although the introduction of three enzymes seems straightforward, the implementation of a heterologous metabolic cycle is challenging as it requires the complete rewiring of the central metabolism of *E. coli.*

In particular, carbon flux through the synthetic autocatalytic RuMP cycle must be tightly coordinated with its effluxes to achieve stable methanol assimilation.

**Rewiring of Central metabolic fluxes**

In previous studies the inventors had generated methanol-dependent strains with a complete RuMP cycle (Keller et al., 2020, https://doi.org/10.1038/s41467-020-19235-5). A particularly promising strain contained two deletions, a deletion in the triose phosphate isomerase (*tpiA*) gene and a deletion in the glutathione-dependent formaldehyde dehydrogenase (*frmA*) gene, abolishing expression of the respective gene products.

The engineered strain *E. coli ΔfrmAΔtpiA* was strictly dependent on methanol for growth and formed about 6.5% of its biomass and about 50% of its RuMP cycle metabolites from the one-carbon substrate.

To establish a fully methylotrophic lifestyle in *E. coli ΔfrmAΔtpiA,* the strain was transformed to express the genes *mdh* (SEQ NO ID 1, 2), *hps* (SEQ NO ID 52, 53), and *phi* (SEQ NO ID 54, 55) from a heterologous plasmid system, as described in the Materials and Methods section hereinunder. This recombinant strain was subsequently subjected to adaptive laboratory evolution (ALE), as described in more detail below. This recombinant *E. coli ΔfrmAΔtpiA* strain, expressing *mdh, hps,* and *phi* is referred to as ancestral *ΔfrmAΔtpiA* strain hereinafter.

Consistent with flux balance analysis (FBA), half of fructose 6-phosphate (F6P) in the ancestral *ΔfrmAΔtpiA* strain was formed from methanol and dihydroxyacetone phosphate (DHAP) originated exclusively from methanol, while metabolites downstream of glyceraldehyde 3-phosphate (GAP) in glycolysis were generated from pyruvate, the second carbon source that was used as a growth substrate.

To evaluate the metabolic adaptations required for achieving growth solely on methanol, the central metabolism of the ancestral *ΔfrmAΔtpiA* strain was modelled by flux variability analysis (FVA), which, in contrast to FBA, allows information regarding the overall solution space that could result in growth on methanol during evolution.

Figure 1A is flux diagram of the predicted distributions of metabolic flux during growth of the ancestral *ΔfrmAΔtpiA* strain on methanol and pyruvate as co-substrates. Figure 1B depicts predicted metabolic flux during growth on methanol alone based on FVA modelling. Metabolic reactions that were synthetically introduced into the metabolism of the ancestral *ΔfrmAΔtpiA* strain and the corresponding enzymes are encircled. The thickness of the lines indicates the strength of the flux. Dashed lines indicate zero flux through the reaction. RuMP cycle, Entner-Doudoroff pathway and the tricarboxylis acid (TCA) cycle are indicated.

Abbreviations on Figure 1 refer to: Mdh, methanol dehydrogenase; Hps, 3-hexulose 6-phosphate synthase; Phi, 6-phospho 3-hexuloisomerase; frmA, S-formylglutathione hydrolase; tpiA, triose phosphate isomerase; CH2O, formaldehyde; H6P, arabino 3-hexulose 6-phosphate; F6P, fructose 6-phosphate; G6P, glucose 6-phosphate; 6PG, 6-phosphogluconate; F1,6bP, fructose 1,6-bisphosphate; S7P, sedoheptulose 7-phosphate; E4P, erythrose 4-phosphate; R5P, ribose 5-phosphate; Ru5P, ribulose 5-phosphate; Xu5P, xylulose 5-phosphate; GAP, glyceraldehyde 3-phosphate; DHAP, dihydroxyacetone phosphate; 1,3bPG, 1,3-bisphosphoglycerate; 3PG, 3-phosphoglycerate; 2PG, 2-phosphoglycerate; PEP, phosphoenolpyruvate.

To allow for a direct comparison of the fluxes, the target growth rate was set to 0.2 h$^{-1}$ in both conditions, a value similar to the growth rate of the native methylotroph *Bacillus methanolicus* at 37°C. The upper bound for the methanol uptake was set to the minimal amount of methanol uptake required (0.6 mmol gCDW$^{-1}$ h$^{-1}$) to achieve the target growth rate in the presence of excess amounts of pyruvate. In the case of growth on methanol alone, the methanol uptake rate was left unbounded.

For growth on pyruvate together with methanol, the highest metabolic fluxes occurred through the tricarboxylic acid (TCA) cycle, while only a small fraction of the total fluxes originated from the RuMP cycle. In contrast, the predicted flux distribution during growth on methanol as the sole carbon source was markedly different.

The fluxes through the TCA cycle were predicted to be reduced to a minimum by a factor of 16, while the fluxes in the RuMP cycle increased about 15-fold.

Furthermore, the Entner-Doudoroff pathway, which was not active under mixed growth conditions, was predicted to be essential for the synthesis of more oxidized metabolites such as pyruvate during sole growth on a C1 compound, such as methanol. Overall, the altered metabolic fluxes confirmed that a complete restructuring of the central metabolism of *E. coli* is required to enable growth on methanol.

To achieve full methylotrophy in the strict sense, a high selection pressure towards more efficient methanol assimilation was applied during the ALE process.

Different from targeted genetic engineering, ALE is an approach to select for random genetic modifications under selective pressure, which can lead to the enrichment of strains with substantial metabolic changes towards a particular trait.

**Long-term evolution of the ancestral *E-coli ΔfrmAΔtpiA* strain**

The ancestral *ΔfrmAΔtpiA* strain was evolved under continuous conditions in a chemostat in which the dilution rate of the culture defined its growth rate.

Figure 2 depicts schematically the targeted metabolic adaptation of the methanol-dependent ancestral *ΔfrmAΔtpiA* strain towards the formation of almost its entire biomass from pyruvate (indicated in white) to growth on methanol alone, with its biomass entirely formed from C1 substrates (indicated in black). The striped part of the cycle indicates a reduced flux.

As shown in Figure 3, during the continuous experiment, methanol was present in excess at a concentration of 500 mM and pyruvate at lower concentration of 20 mM in the chemostat feed which resulted in limiting concentrations of pyruvate (< 0.01 mM) in the growth vessel to ensure a high selection pressure towards increased methanol incorporation. The pyruvate concentration was reduced to 5 mM after 98 generations. The feed medium was supplemented with 0.1 mM isopropyl-β-D-thiogalactopyranosid (IPTG) for heterologous expression of *mdh, hps* and *phi*.

Methanol dehydrogenase 2 (*mdh*) variant CT4-1 from *Cupriavidus necator* was expressed from heterologously introduced plasmid pSEVA424.

3-hexulose 6-phosphate synthase (*hps*) from *Methylobacillus flagellatus* and 6-phospho 3-hexuloisomerase (*phi*) from *Methylobacillus flagellates* were expressed from heterologously introduced plasmid pSEVA131.

Experimental boundary conditions, i.e. the concentrations of methanol and pyruvate in the feed medium and the dilution rate of the culture are shown in the top panel of Figure 3 with observations in terms of the density of the culture over time and number of generations shown in alignment underneath the panel. Due to technical issues, the reactor 1 was restarted after 200 generations (reactor 2) and reactor 2 after 223 generations (reactor 3).

During the first 150 days approximately, which corresponded to $90 \pm 5$ generations based on the selected dilution rate, the optical density of the culture remained rather stable. Then, to further increase the selection pressure towards a higher methanol uptake, the pyruvate concentration was gradually lowered in the feed medium first to 10 mM and ultimately to 5 mM.

While the pyruvate concentration in the growth vessel was already limiting with 20 mM of pyruvate in the feed medium, the reduction to 5 mM should lower the amplitude of the pyruvate fluctuations that arise when a droplet of feed medium enters the growth vessel. As expected, the optical density of the culture proportionally decreased by a factor of four after changing the composition of the feed medium.

A gradual increase in optical density was observed over the subsequent 100 generations. Starting from generation 115 until 223, a steady increase in optical density at 600 nm ($OD_{600}$) from 0.23 to 0.70 was observed, indicating that the population was incorporating more methanol. After another 25 generations and a total of 249 generations, an increase in optical density to 2.5 was observed, indicating another drastic increase in methanol uptake by the population and potentially the feasibility of growth on methanol as the sole carbon and energy source.

## Growth on methanol

The marked increase in yield of the chemostat culture after the long-term evolution of the ancestral *ΔfrmAΔtpiA* strain indicated that the population might be capable of growing on methanol also in the absence of pyruvate. When the population was inoculated in a shake flask with medium containing only methanol (500 mM) as a carbon source, the culture was indeed able to grow, reaching an optical density $OD_{600}$ of about 0.4 at a doubling time ($T_d$) of 60 hours (h), as shown in the growth curve of Figure 4A. In this experiment, growth of the evolved population after 249 generations in the chemostat, in the presence and absence of methanol was examined. The population was grown in minimal medium containing 500 mM methanol, as well as ampicillin and streptomycin.

At this point of the evolution, IPTG, which had been supplied to induce heterologous expression *of mdh, hps* and *phi from* their respective expression vectors, was no longer required for methylotrophic growth.

To improve the growth performance of the population, it was evolved under serial transfer conditions in medium containing only methanol as carbon source.

After 534 generations, 4 individual clones were isolated and characterized. Remarkable the growth rate increased about eightfold in all clones tested ($T_d$ 7.5 $\pm$ 0.8 h), while yields were around $OD_{600}$ 2.1.

Figure 4B shows the growth curve of the best growing isolate, MEcoli_ref_1, grown on methanol under the same conditions. Remarkably, the strain had a doubling time of around eight hours, $T_d$ = 8.1 $\pm$ 0.4 h, as shown in Figure 5B.

Recombinant methylotrophic strain MEcoli_ref_1 was used for all subsequent experiments unless specified otherwise.

## Proteome remodelling in the methylotrophic E. coli

To assess the proteome remodelling of recombinant methylotrophic strain MEcoli_ref_1, the proteome of the methanol-dependent ancestral *ΔfrmAΔtpiA* strain grown on methanol and pyruvate was compared to the proteome of the methylotrophic MEcoli_ref_1 grown on methanol alone.

About 20% of the detected proteins were differentially expressed between the two strains, with 1494 detected proteins, differential expression cutoff | $\log_2$(fold-change) $\geq$ 1.5|.

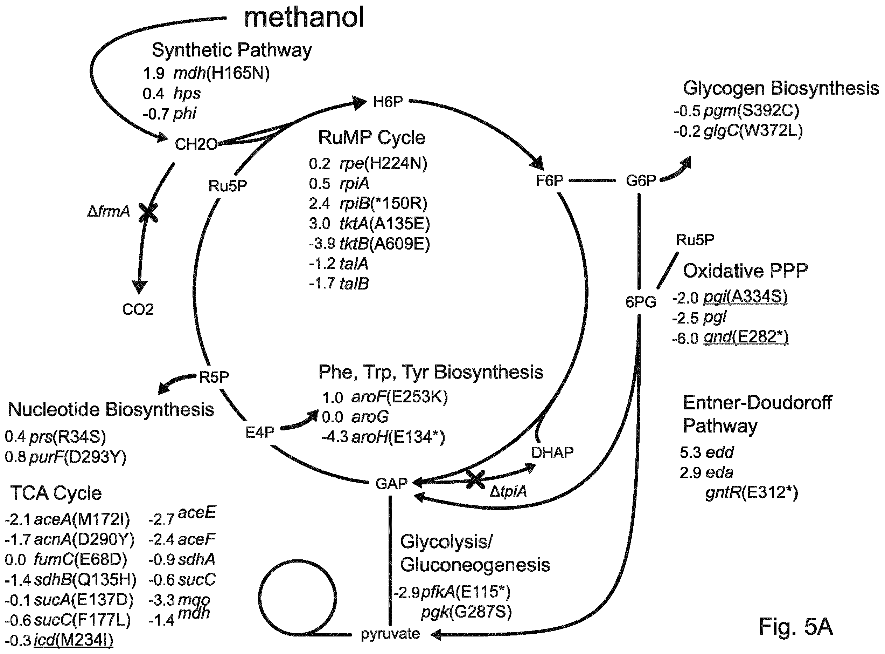

Upon evolution of the ancestral strain to a fully methylotrophic strain, enzymes of the RuMP cycle, the Entner-Doudoroff pathway as well as methanol dehydrogenase were upregulated, while enzymes of branch point reactions away from the RuMP cycle, pyruvate metabolism and the TCA cycle were downregulated, as indicated in Figure 5A.

Figure 5A schematically depicts the mutations in core metabolism identified in the methylotrophic population after 249 generations of chemostat evolution. Additional genetic changes found in MEcoli_ref_1 are underlined in this Figure. Difference in expression levels ($\log_2$(fold-change)) based on proteomics data are shown to the left of the corresponding gene name.

Figure 5B is a comparison chart of differential gene expression, comparing the ancestral *ΔfrmAΔtpiA E. coli* strain grown in minimal medium supplemented with 500 mM methanol and 20 mM pyruvate to MEcoli_ref_1 grown in medium containing only 500 mM methanol as carbon source. Q values are P values, indicating the probability that the observed result occurred by chance, adjusted for multiple hypothesis testing using the Benjamini-Hochberg procedure. Black symbols indicate differentially expressed genes (log2(fold-change) $\geq$ 1.5) of important metabolic pathways and the enriched supergroup of DNA repair and replication. The latter is a superset of the KEGG pathways mismatch repair (eco03430), DNA replication (eco03030) and homologous recombination (eco03440). Results are also summarised in Table 1.

In addition, the proteome data were contextualised by estimating relative abundances of individual proteins, which revealed that methanol dehydrogenase increased in abundance from 16% of the quantifiable proteome in the ancestral *ΔfrmAΔtpiA E. coli* strain to 40% in MEcoli_ref_1.

To find additional cellular functions with altered expression profiles, gene set enrichment analysis on KEGG pathway annotations were performed. Interestingly, the analysis showed that proteins related to DNA replication, mismatch repair and homologous recombination were overrepresented in the evolved strain (Table 1).

**Table 1:** Differences in expression of relevant genes in $\log_2$ fold-change, "$\log_2$FC", as well as the pathways in which these genes are implicated, are listed.

| KEGG pathway | gene | $\log_2$FC |
|---|---|---|
| Homologous recombination | holA | 3.40 |
| Homologous recombination | priB | 3.24 |
| Homologous recombination | recD | 2.17 |
| Homologous recombination | dnaE | 1.96 |
| Homologous recombination | recA | 1.65 |
| Homologous recombination | recG | 1.61 |
| Homologous recombination | ruvB | 1.44 |
| Homologous recombination | holD | 1.43 |
| Homologous recombination | ruvA | 1.26 |
| Homologous recombination | dnaN | 1.26 |
| Homologous recombination | dnaX | 1.16 |
| Citrate cycle (TCA cycle) | sdhA | -0.93 |
| Citrate cycle (TCA cycle) | sdhB | -1.35 |
| Citrate cycle (TCA cycle) | mdh | -1.40 |
| Citrate cycle (TCA cycle) | sdhD | -1.55 |
| Citrate cycle (TCA cycle) | gltA | -1.60 |
| Citrate cycle (TCA cycle) | acnA | -1.73 |
| Citrate cycle (TCA cycle) | frdA | -2.02 |
| Citrate cycle (TCA cycle) | aceF | -2.36 |
| Citrate cycle (TCA cycle) | aceE | -2.69 |
| Citrate cycle (TCA cycle) | frdB | -3.00 |
| Citrate cycle (TCA cycle) | mqo | -3.34 |
| Pentose and glucuronate interconversions | ugd | 3.52 |
| Pentose and glucuronate interconversions | uxuA | 2.31 |
| Pentose and glucuronate interconversions | xylA | 2.26 |
| Pentose and glucuronate interconversions | xylB | 2.24 |
| Pentose and glucuronate interconversions | rspA | 1.90 |
| Pentose and glucuronate interconversions | uxuB | 1.83 |
| Pentose and glucuronate interconversions | uxaC | 1.37 |
| Pentose and glucuronate interconversions | yagE | 1.22 |
| Galactose metabolism | melA | -2.22 |
| Galactose metabolism | gatB | -2.78 |
| Galactose metabolism | gatA | -2.80 |
| Galactose metabolism | pfkA | -2.91 |
| Galactose metabolism | gatD | -2.93 |
| Galactose metabolism | gatY | -3.00 |
| Galactose metabolism | gatZ | -3.66 |

(continued)

| KEGG pathway | gene | log$_2$FC |
|---|---|---|
| Pyruvate metabolism | adhE | -1.18 |
| Pyruvate metabolism | ldhA | -1.29 |
| Pyruvate metabolism | gloB | -1.29 |
| Pyruvate metabolism | mdh | -1.40 |
| Pyruvate metabolism | poxB | -1.49 |
| Pyruvate metabolism | ackA | -1.50 |
| Pyruvate metabolism | hchA | -1.59 |
| Pyruvate metabolism | pflB | -1.80 |
| Pyruvate metabolism | yahK | -1.98 |
| Pyruvate metabolism | frdA | -2.02 |
| Pyruvate metabolism | yccX | -2.10 |
| Pyruvate metabolism | aceF | -2.36 |
| Pyruvate metabolism | aceE | -2.69 |
| Pyruvate metabolism | frdB | -3.00 |
| Pyruvate metabolism | glcB | -3.04 |
| Pyruvate metabolism | mqo | -3.34 |
| Pyruvate metabolism | adhP | -4.48 |
| Histidine metabolism | hisC | 2.09 |
| Histidine metabolism | hisA | 1.60 |
| Histidine metabolism | hisB | 1.53 |
| Histidine metabolism | hisG | 1.33 |
| Histidine metabolism | hisI | 1.28 |
| Histidine metabolism | hisD | 1.27 |
| Histidine metabolism | hisF | 1.24 |
| Histidine metabolism | hisH | 1.00 |
| DNA replication | holA | 3.40 |
| DNA replication | dnaE | 1.96 |
| DNA replication | holD | 1.43 |
| DNA replication | dnaN | 1.26 |
| DNA replication | dnaX | 1.16 |
| Mismatch repair | holA | 3.40 |
| Mismatch repair | dnaE | 1.96 |
| Mismatch repair | holD | 1.43 |
| Mismatch repair | uvrD | 1.39 |
| Mismatch repair | dnaN | 1.26 |
| Mismatch repair | mutS | 1.19 |
| Mismatch repair | dnaX | 1.16 |
| Mismatch repair | xseA | 0.98 |

(continued)

| KEGG pathway | gene | log$_2$FC |
|---|---|---|
| Mismatch repair | mutL | 0.81 |

**Evolutionary trajectory towards full methylotrophy and genetic modifications**

To identify genetic changes acquired during evolution towards full methylotrophy, the ancestral $\Delta frmA\Delta tpiA$ *E. coli* strain and the fully methylotrophic reference strain MEcoli_ref_1 were sequenced. In addition, the metagenomic composition of the evolving population was determined at regular time intervals to obtain temporal information of the adaptation process.

After 52 generations the number of observed and fixed mutations, i.e. mutations of an abundance greater than 90% in the population, started to increase. After 249 generations, when the population achieved methylotrophic growth in the chemostat, 564 mutations had fixed in the population.

Subsequent evolution under serial dilution regime further increased this number to about 1000. Ultimately, clones isolated after 534 generations, the same ones for which growth rates were determined, acquired on average 1155 ± 52 (mean ± standard deviation) mutations. MEcoli_ref_1 carried 1231 mutations of which 55% (677 of 1231) were nonsynonymous.

To detect patterns in the evolution towards full methylotrophy, the sequence in which metabolic adaptations in methanol dehydrogenase, the RuMP cycle, RuMP cycle efflux points and the Entner-Doudoroff pathway arose in the population was further explored.

Results of this analysis are summarised in Table 2.

Mutations in TCA and RuMP cycle genes dominated the adaptation process during the first 150 generations and were followed by the occurrence of a mutation in methanol dehydrogenase. In the last phase before full methylotrophy was achieved, many mutations in the analyzed metabolic pathways fixed in the population.

Particularly, the only mutation relevant of the Entner-Doudoroff pathway (*gntR*(E312*)) occurred at this timepoint. Over the course of the serial dilution, another selective sweep occurred, fixing two mutations in RuMP cycle efflux points. Interestingly, despite the higher growth rate of MEcoli_ref_1, the core set of metabolic adaptations as shown in Figure 5A was not expanded drastically over continued evolution under serial dilution regime.

Besides the core methanol metabolization pathways, a search for other functional classes that were altered during the course of evolution was conducted, in particular for KEGG pathways that were enriched in nonsynonymous mutations in MEcoli_ref_1. No significant hits were identified.

As shown in Figure 8, several of metabolites were detected in the growth supernatant of MEcoli_ref_1 when grown in minimal medium supplied with 500mM of methanol under ambient conditions. Formate and deoxyribose were the most abundant natural metabolites produced and excreted by this strain.

The impact of the mutation in the key enzyme for full methylotrophy, i.e. methanol dehydrogenase was examined more closely. The mutation H165N in the Mdh CT1-4 polypeptide resulted in an about two-fold increase in its catalytic turnover number (Figure 9).

**Table 2:** Mutations and Proteomics data of recombinant methylotroph MEcoli_ref_1

| Gene | Encoded protein name | log$_2$(FC) expression level | Mutation | Generation | (presumed) effect on activity | SEQ ID NO |
|---|---|---|---|---|---|---|
| *mdh* | *Cupriavidus necator* N-1 methanol dehydrogenase 2 | 1.9 | H165N | 224 | increased catalytic turnover | 1,2 |
| *rpe* | Ribulose-phosphate 3-epimerase | 0.2 | H224N | 142 | presumed increase in activity | 3,4 |
| *rpiB* | Ribose-5-phosphate isomerase B | 2.4 | *150R | 86 | presumed increase in activity | 5,6 |
| *tktA* | Transketolase 1 | 3 | A135E | 249 | presumed increase in activity | 7, 8 |

(continued)

| Gene | Encoded protein name | log$_2$(FC) expression level | Mutation | Generation | (presumed) effect on activity | SEQ ID NO |
|------|---------------------|------------------------------|----------|------------|-------------------------------|-----------|
| tktB | Transketolase 2 | -3.9 | A609E | 249 | presumed increase in activity | 9, 10 |
| pgi | Glucose-6-phosphate isomerase | -2 | A334S | 434 | presumed decrease in activity | 11, 12 |
| gnd | 6-phosphogluconate dehydrogenase | -6 | E282* | 434 | presumed loss of activity | 13, 14 |
| gntR | DNA-binding transcriptional repressor GntR | NA | E312* | 249 | presumed loss of repression | 15, 16 |
| pfkA | 6-phosphofructokinase 1 | -2.9 | E115* | 78 | presumed loss of activity | 17, 18 |
| pgk | Phosphoglycerate kinase | -0.5 | G287S | 249 | presumed decrease in activity | 19, 20 |
| aroF | 3-deoxy-7-phosphoheptulonate synthase | 1 | E253K | 52 | presumed decrease in activity | 21, 22 |
| aroH | 3-deoxy-7-phosphoheptulonate synthase | -4.3 | E134* | 249 | presumed decrease in activity | 23, 24 |
| prs | ribose-phosphate diphosphokinase | 0.4 | R34S | 249 | presumed decrease in activity | 25, 26 |
| purF | Amidophosphoribosyltransferase | 0.8 | D293Y | 56 | presumed decrease in activity | 27, 28 |
| aceA | isocitrate lyase | -2.1 | M172I | 218 | presumed decrease in activity | 29, 30 |
| acnA | aconitate hydratase A | -1.7 | D290Y | 218 | presumed decrease in activity | 31, 32 |
| fumC | fumarase C | 0 | E68D | 249 | presumed decrease in activity | 33, 34 |
| sdhB | succinate:quinone oxidoreductase | -1.4 | Q135H | 249 | presumed decrease in activity | 35, 36 |
| sucA | 2-oxoglutarate decarboxylase | -0.1 | E137D | 60 | presumed decrease in activity | 37, 38 |
| sucC | succinyl-CoA synthetase subunit β | -0.6 | F177L | 60 | presumed decrease in activity | 39, 40 |

(continued)

| Gene | Encoded protein name | log$_2$(FC) expression level | Mutation | Generation | (presumed) effect on activity | SEQ ID NO |
|---|---|---|---|---|---|---|
| *icd* | isocitrate dehydrogenase | -0.3 | M234I | 534 | presumed decrease in activity | 41, 42 |
| *crp* | DNA-binding transcriptional dual regulator CRP | 0.4 | E97D | 142 | presumed global remodelling of proteome | 43, 44 |
| *rpoB* | RNA polymerase subunit β | 0.5 | Q658L | 224 | presumed global remodeling of proteome | 45, 46 |
| *rpoC* | RNA polymerase subunit β' | 0.4 | D516Y | 86 | Presumed global remodeling of proteome | 47, 48 |
| *rpoC* | RNA polymerase subunit β' | 0.4 | R1231L | 249 | Presumed global remodeling of proteome | 47, 48 |
| 3075114 | | 3 | C -> A | | Presumed increase in *tktA* expression | 51 |

The column "Gene" lists the name of the mutated gene of, or the genome location in the ancestral strain BW25113 (Genbank accession: NZ_CP009273). The numbers listed in column "Generation" indicate the number of generations during ALE in which the mutation was fixed. "SEQ ID NO" refers to the sequence identity number of the sequence listing filed separately for this invention.

The above-listed mutations in the genes *rpe, rpiB, tktA* and *tktB* presumably increase the metabolic flux through the RuMP cycle.

The above-listed mutations in the genes *pgi, aroF, aroH, prs,* and *purF,* presumably stabilize the metabolic flux through the RuMP cycle.

The above-listed mutations in the genes *pgi, aroF, aroH, prs,* and *purF,* presumably stabilize the metabolic flux through the RuMP cycle.

The above-listed mutations in the genes *aceA, acnA, fumC, sdhB, sucA* and *icd* presumably decrease the flux through the TCA cycle.

The above-listed mutations in genes encoding the master regulators *crp, rpoB* and *rpoC* presumably cause these regulators to remodel the proteome to enable methylotrophic growth.

The above-listed mutation in the *gnd* gene results in a truncation of the 6-phosphogluconate dehydrogenase polypeptide, which removes the substrate binding site of the enzyme and therefore presumably abolishes its activity. This prevents flux from the Entner-Doudoroff pathway intermediate 6-phospho-D-gluconate to D-ribulose 5-phosphate under loss of $CO_2$.

The above-listed mutation in the *gntR* gene results in a truncation of the DNA-binding transcriptional repressor GntR, removing a part of its effector binding and oligomerization domain. Presumably this prevents dimerization of the repressor and, in turn, repression of the Entner-Doudoroff pathway genes *edd, eda.* These genes are highly upregulated in MEcoli_ref_1.

The above-listed mutation in the *pfkA* gene removes the substrate binding site of the 6-phosphofructokinase 1 enzyme and presumably abolishes its activity. This prevents flux from the RuMP cycle into the metabolic dead end metabolite fructose 1,6-bisphosphate which cannot be further metabolized due to the *tpiA* deletion in MEcoli_ref_1.

The above-listed mutation in the *pgk* gene presumably decreases its activity such that flux coming out of the Entner-Doudoroff pathway is directed towards the TCA cycle and not back towards D-glyceraldehyde 3-phosphate and with that back into the RuMP cycle under loss of ATP.

The above listed mutation of the gene encoding the *Cupriavidus necator* N-1 methanol dehydrogenase 2 Mdh increases catalytic turnover number of its gene product, methanol dehydrogenase, two-fold. The increased catalytic activity of this enzyme enables faster methanol oxidation.

**Proteomic and genetic modifications to achieve full methylotrophy**

To enable growth on methanol, the metabolism of the methanol-dependent ancestral *ΔfrmAΔtpiA* had to be globally rewired to achieve full methylotrophy. The observed changes concerned in methanol oxidation, the RuMP cycle, the Entner-Doudoroff pathway and the TCA cycle.

The recombinant methylotrophic microorganism of this invention does not express a polypeptide having glutathione-dependent formaldehyde dehydrogenase FrmA activity and a polypeptide having triose phosphate isomerase TpiA activity.

In addition, the recombinant methylotrophic microorganism of this invention expresses or over-express, when compared to its ancestral non-fully methylotrophic microorganism, polypeptide having methanol dehydrogenase activity, such as Mdh, a polypeptide having 3-hexulose-6-phosphate activity, such as Phi, and polypeptide having a 6-phospho 3-hexuloisomerase activity, such as Hps.

This invention identifies further genes which, when modified or expressed differently compared to a non-methylotrophic parental strain, may positively contribute to achieving full methylotrophy in a non-naturally occurring methylotroph.

In one embodiment of this invention a recombinant methylotrophic microorganism expresses or over-expresses, as compared to its ancestral non-methylotrophic microorganism, one or more polypeptides selected from the group consisting of Mdh, Hps, Phi, TktA, TktB, Rpe, RpiA, RpiB, TalA, TalB, Edd, and Eda, or any combination thereof.

A recombinant methylotrophic microorganism of this invention may include increased activity of expression of a polypeptide having ribulose-phosphate 3-epimerase (Rpe) activity and which shares at least 40%, 45%, 50%, 55%, 60%, 6%, 70%, 75%, 80%, 85%, 90%, 95%, 96%, 97%, 98%, 99% (or a value between any two of the foregoing values) or greater sequence identity, or at least 50%, 60%, 70%, 80%, 90%, 95%, 96%, 97%, 98%, 99% (or a value between any two of the foregoing values) or greater sequence similarity, as calculated by NCBI BLAST, using default parameters, to SEQ ID NO 3. Preferably the polypeptide comprises at least a H224N mutation compared to the wild-type Rpe polypeptide.

A recombinant methylotrophic microorganism of this invention may include increased activity of expression of a polypeptide having Ribose-5-phosphate isomerase B (RpiB) activity and which shares at least 40%, 45%, 50%, 55%, 60%, 6%, 70%, 75%, 80%, 85%, 90%, 95%, 96%, 97%, 98%, 99% (or a value between any two of the foregoing values) or greater sequence identity, or at least 50%, 60%, 70%, 80%, 90%, 95%, 96%, 97%, 98%, 99% (or a value between any two of the foregoing values) or greater sequence similarity, as calculated by NCBI BLAST, using default parameters, to SEQ ID NO 5. Preferably the polypeptide comprises at least a *150R mutation compared to the wild-type RpiB polypeptide.

A recombinant methylotrophic microorganism of this invention may include increased activity of expression of a polypeptide having Transketolase 1 (TktA) activity and which shares at least 40%, 45%, 50%, 55%, 60%, 6%, 70%, 75%, 80%, 85%, 90%, 95%, 96%, 97%, 98%, 99% (or a value between any two of the foregoing values) or greater sequence identity, or at least 50%, 60%, 70%, 80%, 90%, 95%, 96%, 97%, 98%, 99% (or a value between any two of the foregoing values) or greater sequence similarity, as calculated by NCBI BLAST, using default parameters, to SEQ ID NO 7. Preferably the polypeptide comprises at least a A135E mutation compared to the wild-type TktA polypeptide.

A recombinant methylotrophic microorganism of this invention may include increased activity of expression of a polypeptide having phosphogluconate dehydratase (Edd) activity.

A recombinant methylotrophic microorganism of this invention may include increased activity of expression of a polypeptide having 2-keto-3-deoxygluconate 6-phosphate/2-keto-4-hydroxyglutarate aldolase activity (Eda) activity.

A recombinant methylotrophic microorganism of this invention may include increased activity of expression of a polypeptide having TktA activity and a polypeptide having RpiB activity, or of a polypeptide having TktA activity and a polypeptide having Edd activity and/or a polypeptide having Eda activity, or of a polypeptide having RpiB and a polypeptide having Edd activity and/or polypeptide having Eda activity.

In another or further embodiment, the recombinant methylotroph may comprise one or more modifications in one or more native genes increasing the enzymatic activity of the polypeptide encoded by the mutated gene compared to the polypeptide encoded by the parental gene.

A recombinant methylotrophic microorganism of this invention may for example comprise modifications increasing activity of polypeptides encoded by one or more native genes selected from the group consisting of *rpe, rpiB, tktA,* and *tktB,* or any combination thereof.

A recombinant methylotrophic microorganism of this invention may have Rpe polypeptide having a sequence identity that is at least 95% to 100% identical to SEQ ID NO 4. Preferably, residue 224 of the Rpe polypeptide is an asparagine.

A recombinant methylotrophic microorganism of this invention may have RpiB polypeptide having a sequence identity that is at least 95% to 100% identical to SEQ ID NO 6. Preferably, residue 150 of the RpiB polypeptide is an arginine.

A recombinant methylotrophic microorganism of this invention may have TktA polypeptide having a sequence identity that is at least 95% to 100% identical to SEQ ID NO 8. Preferably, residue 135 of the TkTA polypeptide is a glutamate.

A recombinant methylotrophic microorganism of this invention may have TktB polypeptide having a sequence identity that is at least 95% to 100% identical to SEQ ID NO 10. Preferably, residue 609 of the TkTB polypeptide is a glutamate.

In another or further embodiment, a recombinant methylotrophic microorganism of this invention may comprise modifications decreasing or eliminating activity of polypeptides encoded by one or more native genes selected from the group consisting *of pgi, gnd, gntR, pfkA, pgk, aroF, aroH, prs, purF, aceA, acnA, fumC, sdhB, sucA, sucC,* and *icd,* or any combination thereof.

A recombinant methylotrophic microorganism of this invention may comprise a Glucose-6-phosphate isomerase Gpi activity, or a phosphoglycerate kinase Pgk activity, or a 3-deoxy-7-phosphoheptulonate synthase aroF activity, or a 3-deoxy-7-phosphoheptulonate synthase aroH activity, or a ribose-phosphate diphosphokinase Prs activity, or a amidophosphoribosyltransferase PurF activity, or a isocitrate lyase AceA activity, or a aconitate hydratase A AcnA activity, or a fumarase C FumC activity, or a succinate:quinone oxidoreductase SdhB activity, or a 2-oxoglutarate decarboxylase SucA activity, or a succinyl-CoA synthetase subunit β Suc C activity, or a isocitrate dehydrogenase Icd activity that is 40% or less, for example 20%, 25%, 30%, 35%, 40% (or a value between any two of the foregoing values), of the enzymatic activity of the respective wild-type polypeptide. A recombinant methylotrophic microorganism of this invention may comprise any reduction of activity of any combination of the polypeptides listed here.

A recombinant methylotrophic microorganism of this invention may comprise a loss of 6-phosphogluconate dehydrogenase Gnd activity, or a 6-phosphogluconate dehydrogenase Gnd activity that is 40% or less, for example 20%, 25%, 30%, 35%, 40% (or a value between any two of the foregoing values), of the enzymatic activity of the respective wild-type polypeptide.

A recombinant methylotrophic microorganism of this invention may comprise a loss of 6-phosphofructokinase 1 Pfk1 activity, or a 6-phosphofructokinase 1 Pfk1 activity that is 40% or less, for example 20%, 25%, 30%, 35%, 40% (or a value between any two of the foregoing values), of the enzymatic activity of the respective wild-type polypeptide.

A recombinant methylotrophic microorganism of this invention may comprise a loss of DNA-binding transcriptional repressor GntR activity, or a DNA-binding transcriptional repressor GntR activity that is 40% or less, for example 20%, 25%, 30%, 35%, 40% (or a value between any two of the foregoing values), of the enzymatic activity of the respective wild-type polypeptide.

The loss of activity of the GntR repressor may be due to a modification in the *gntR* gene preventing the dimerization of the repressor. The modification may for example be a mutation resulting in the truncation of the GntR repressor. The modification may result in an at least partial deletion of the oligomerization domain of the GntR repressor. The mutation may also alter or delete at least parts of the effector binding domain of the GntR repressor, such that the repressor no longer responds to the effector molecule.

In yet another or further embodiment, a recombinant methylotrophic microorganism of this invention may comprise further modifications to reduce or eliminate the expression of one or more polypeptides from native genes encoding an enzyme of the TCA cycle, including *aceA, acnA, fumC, sdhB, sucA, sucC, icd, aceE, aceF, sdhA, sucC,* and *mqo, or* any combination thereof.

A recombinant methylotrophic microorganism of this invention may for example comprise a reduction in expression, or a deletion of the gene encoding the Gnd polypeptide and in the gene encoding the AroH polypeptide.

Polynucleotide sequences encoding polypeptides can be derived from the relevant nucleotide sequences by using well known codon tables and the degeneracy of the genetic code. Nucleotide sequences are provided in the sequence listings as identified by their sequence identity number (SEQ ID NO).

The introduction of only two mutations (*ΔfrmA, ΔtpiA*) together with the heterologous expression of three genes, was sufficient to generate an *E. coli* strain growing on methanol after about 250 generations in a continuous chemostat culture. The evolved strain builds its entire biomass from the reduced one-carbon compound, as we demonstrate by metabolic tracer experiments, and grows at a doubling time of about 8 hours.

**Biomass and metabolite formation from methanol**

To confirm complete biomass formation from methanol alone, $^{13}C$ metabolic tracer analysis was used. The results of these assays are shown in Figures 6A to 6D.

Figures 6A and 6D show the $^{13}$C labelled fraction of protein-bound amino acids by liquid chromatography coupled mass spectrometry (LC-MS) in MEcoli_ref_1 grown in medium containing no additional carbon sources. Antibiotics, IPTG and EDTA were omitted from the minimal medium in these studies to ensure that methanol and $CO_2$ were the only available carbon sources for growing the strain. Error bars represent the standard deviation for three technical replicates of MEcoli_ref_1.

Isotopologue distributions, shown in Figures 6A and 6C, and fractional contributions, shown in Figures 6B and 6D, were determined for different protein bound amino acid by LC-MS.

For isotopologue distributions, n refers to the fully labelled molecule and n-1, n-2, etc. to molecules with one, respectively two, etc., unlabelled carbon atoms.

When grown under ambient atmosphere, appreciable amounts of $^{12}$C label present in protein-bound amino acids were observed (Figure 6A, 6B). Due to the absence of additional carbon sources in the medium, the remaining source of $^{12}$C carbon was ambient $CO_2$. Consequently, when MEcoli_ref_1 was grown under an atmosphere enriched to 5% (V/V) [13]$CO_2$, protein-bound amino acids were fully labelled (Figure 6C and 6D).

In Figures 7A to 7D a similar labelling pattern is shown for extracted metabolites of methylotrophic clones from an earlier stage of the serial dilution evolution. In addition, the total biomass labelling ratio using elemental analyser/isotope ratio mass spectrometry is shown.

Label incorporation was studied in four replicates isolated from the evolving serial dilution experiment after 384 generations. Cultures were grown in minimal medium supplemented with 500 mM $^{13}$C methanol either at ambient $CO_2$, as shown in Figures 7A and B, or at 5% (V/V) enriched [13]$CO_2$ atmosphere, as shown in Figures 7C and D.

Antibiotics, IPTG and EDTA were omitted out from the minimal medium to ensure that methanol and $CO_2$ are the only available carbon sources. The isotopologue distribution, as shown in Figures 7A and 7C, and fractional contribution, as shown in Figures 7B and 7D, was determined for different central metabolites by LC-MS. The central metabolites measured were hexose phosphates (H6P), pentose phosphates (P5P), sedoheptulose 7-phosphate (S7P), 2-phosphoglycerate/3-phosphoglycerate (2PG/3PG), asparagine (Asn), arginine (Arg), glutamine (Gln), and lysine (Lys).

For technical reasons the different hexose phosphates (glucose 6-phosphate, fructose 6-phosphate, arabino 3-hexulose 6-phosphate), pentose phosphates (ribose 5-phosphate, ribulose 5-phosphate, xylulose 5-phosphate), and 2-phosphoglycerate and 3-phosphoglycerate were summarized as one group. S7P was detected in only 3 out of 4 replicates in the [12]$CO_2$ condition.

For the isotopologue distribution, n refers to the fully labelled molecule and n-1, n-2, etc to molecules with one, respectively two, unlabelled carbon atoms.

Error bars represent the standard deviation for four biological replicates for the [12]$CO_2$ condition and for three for the [13]$CO_2$ condition.

When grown under ambient atmosphere, 83.9 $\pm$ 4.6% (mean $\pm$ standard deviation) of the total biomass of MEcoli_ref_1 was $^{13}$C labelled. We found that this matches the FBA model that predicts total biomass labelling of about 83%.

When MEcoli_ref_1 was grown under [13]$CO_2$ enriched atmosphere 98.6 $\pm$ 0.3% mean $\pm$ standard deviation of its biomass was derived from $^{13}$C, as expected.

Lastly, the $^{13}$C fraction of protein-bound amino acids (Figure 4B) allowed to discern where $CO_2$ entered metabolism. $CO_2$ did not contribute to the biosynthesis of amino acids directly derived from the RuMP cycle (His, Phe, Tyr) and only little to ones (Ala, Leu, Ser, Val) with gluconeogenic precursors. Noticeably higher levels of $CO_2$ incorporation were found in amino acids derived from the TCA cycle (Arg, Asp, Glu, Ile, Pro, Thr).

These metabolic tracer experiments proved, that the evolved strain builds its entire biomass from the reduced one-carbon compound. The evolved *E. coli* strain grows at a doubling time of about 8 hours on the reduced one-carbon compound.

The recombinant methylotroph proposed by this invention provides a valuable tool for microbial conversion of reduced C1 compounds, in particular methanol, into value-added compounds and for applications in industrial biotechnology.

**A recombinant Methanol dehydrogenase**

The ancestral strain used for generating the fully methylotrophic *E. coli* MEcoli_ref_1 strain according to this disclosure transformed to heterologously produce the methanol dehydrogenase 2 (Mdh) variant CT4-1 from *Cupriavidus necator,* SEQ ID NO 1, as described herein.

The ancestral Mdh variant CT4-1 has been described previously by Wu, T.-Y. et al., 2016, Appl. Microbiol. Biotechnol. 100, 4969-4983; DOI: 10.1007/s00253-016-7320-3.

Remarkably, following the long-term evolution study, a point mutation of the mdh gene could be identified which resulted in a significantly increased activity of the modified Mdh polypeptide (SEQ ID NO 58, 59). The point mutation caused an amino acid change H165N in the enzyme.

The mutated Mdh H165N polypeptide was isolated, and its activity was tested *in vitro* as described in the Material and Methods section.

To this end, the ancestral *mdh* CT4-1 sequence and the mutant mdh H165N sequence encoding the respective methanol dehydrogenases were cloned into a commercially available pET16b expression vector using Gibson assembly. The resulting constructs were His-tagged and were purified using nickel-immobilized metal affinity chromatography.

Catalytic activity of both enzymes was then measured in *in vitro* assays, and the results of these assays are shown in Figure 9. Maximum reaction speed $v_{max}$ was determined for purified His-tagged Mdh and Mdh(H165N) on the basis of measured increase in fluorescent counts indicated in arbitrary units per second (a.u./s). Equal amounts of protein were used in both assays and methanol turnover measured by following formation of $NADH/H^+$ in a spectrophotometer, i.e. absorbance increase at 340 nm over time.

It was established as part of this disclosure, that a Mdh polypeptide bearing this mutation has an improved methanol dehydrogenase activity compared to the parental CT4-1 variant of C. *necator* consisting of SEQ ID NO: 1. By introducing an H165N mutation in a Mdh polypeptide, the methanol dehydrogenase catalytic turnover number compared to the parental Mdh polypeptide can be increased by at least 1.5 fold, or at least 2-fold, or at least 2.5-fold.

This disclosure also concerns a modified polypeptide having at least 70%, 80%, 90%, 95%, 98% or 99% sequence identity to SEQ ID NO: 1 and comprises at least an H165N mutation.

Preferably, the modified polypeptide is engineered from a Mdh polypeptide *of C. necator,* or from the engineered CT4-1 Mdh variant of C. *necator.*

The modified polypeptide of this disclosure preferably has an improved methanol dehydrogenase activity compared to its ancestral Mdh polypeptide, for example the CT4-1 variant of C. *necator.*

The modified polypeptide may have an increased reaction rate $v_{max}$ of at least 1.2-fold greater, at least 1.5-fold, at least 1.8-fold, at least 2-fold, at least 2.2-fold, at least 2.5-fold. at least 2.8-fold, or at least 3-fold greater than its parental polypeptide.

The disclosure furthermore concerns an isolated nucleic acid encoding the modified polypeptide.

The modified polypeptide may be expressed from a plasmid, preferably from an expression vector.

The modified polypeptide may be overexpressed for further processing, such as purification.

The modified polypeptide may also be expressed in a recombinant host cell to introduce or to enhance a certain metabolic trait of said microorganism.

The expression modified polypeptide in the recombinant host cell, may result in the production of a metabolite of product by the recombinant host cell, which it would not normally produce. The expression modified polypeptide may also result in an increase of production of such a metabolite or product when compared to the wild-type host cell.

The expression modified polypeptide in the recombinant host cell, may result in introducing or enhancing the capability of the recombinant host cell to metabolise a substrate, such as methanol or another C1 compound, when compared to the parental host cell.

The modified polypeptide may be expressed in a host cell. The host cell may be a eukaryotic cell or a prokaryotic cell.

The parental host cell may for example be a non-naturally occurring recombinant methylotrophic microorganism. The parental host cell may for example be the *ΔfrmAΔtpiA* strain.

## Materials and Methods

### Reagents and media:

Chemicals were obtained from Sigma-Aldrich Chemie GmbH, Buchs, Switzerland unless otherwise specified. The M9 minimal medium used for bacterial cultivation consisted of the following salts (g L$^{-1}$): $Na_2HPO_4$ (6.78), $KH_2PO_4$ (3.0), NaCl (0.5), $NH_4Cl$ (1.0), $CaCl_2$ (0.735), $MgSO_4$ (0.123) and trace elements. Trace elements were present in the medium at the following concentrations (mg L$^{-1}$): $Na_2EDTA$ (5.0), $MnSO_4$ (5.0), $FeSO_4 \cdot 7\ H_2O$ (1.0), $CO(NO_3)_2 \cdot 6H_2O$ (1.0), $ZnSO_4 \cdot 7\ H_2O$ (1.0), $CuSO_4 \cdot 5\ H_2O$ (0.1), $Na_2MoO_4 \cdot 2\ H_2O$ (0.1), $NiCl_2 \cdot 6\ H_2O$ (0.2). If indicated, antibiotics were added in the following concentrations (mg L$^{-1}$): ampicillin (100), carbenicillin (50), streptomycin sulfate (20).

### Primers and plasmids used in this study

Plasmids and primers used in this study are listed in Tables 3 and 4. The heterologously introduced plasmids were pSEVA424 with the methanol dehydrogenase 2 (*mdh*) gene variant CT4-1 from *Cupriavidus necator,* as described in Wu, T.-Y. et al., 2016, Appl. Microbiol. Biotechnol. 100, 4969-4983; DOI: 10.1007/s00253-016-7320-3, and pSEVA131 with the 3-hexulose 6-phosphate synthase (*hps*) and the 6-phospho 3-hexuloisomerase (*phi*) from *Methylobacillus flagellatus.* The nucleotide sequence of the plasmids was confirmed by PCR and Sanger sequencing (Microsynth AG; Switzerland).

**Table 3:** Plasmids used in this study

| Plasmid | Antibiotic resistance | Description | SEQ ID NO | Reference |
|---|---|---|---|---|
| pSEVA131 *hps phi Methylobacillus flagellatus* | Streptomycin | vector expressing 3-hexulose 6-phosphate synthase and 6-phospho 3-hexuloisomerase for formaldehyde to fructose 6-phosphate conversion | 56 | Keller P. et al. Methanol-dependent Escherichia coli strains with a complete ribulose monophosphate cycle. Nat. Commun. 11, 1-10 (2020). |
| pSEVA424 *mdh2* CT4-1 *Cupriavidus necator* | Ampicillin | vector expressing methanol dehydrogenase for methanol to formaldehyde conversion | 57 | Meyer, F. et al. Methanol-essential growth of Escherichia coli. Nat. Commun. 9, (2018). |

**Table 4:** Oligonucleotides used in this study

| Primer | SEQ NO ID | Sequence | Description |
|---|---|---|---|
| frmA_fw | 60 | GAAAGCCATATCCGGGAAAC | Verification of *frma* knockout |
| frmA rv - | 61 | GACGCCGACATTCATTTCAC | Verification of *frma* knockout |
| tpiA_fw | 62 | GGGCGGCCATCTTCCTTTATTC | Verification of *tpiA* knockout |
| tpiA_rv | 63 | GGCGACACAACGAAATCTACTG | Verification of *frma* knockout |
| pSEVA_seq_fw | 64 | CATCCGGCTCGTATAATGTG | Verification of pSEVA424 mdh2 CT4-1 C. necator |
| pSEVA_seq_rv | 65 | GAGTTCTGAGGTCATTACTG | Verification of pSEVA424 |
| | | | mdh2 CT4-1 C. necator |

Strains used in this study

The starting strain of the evolution experiment *E. coli* BW25113 *ΔfrmAΔtpiA* containing pSEVA424 *mdh C. necator* (SEQ NO ID 1, 2), and pSEVA131 *hps* (SEQ NO ID 52, 53) and *phi M.flagellatus* (SEQ NO ID 54, 55) was described in a previous study, as disclosed in Keller, P. et al., 2020, Nat. Commun. 11, 5403, https://doi.org/10.1038/s41467-020-19235-5.

**Table 5:** *E. coli* strains used in this study

| Strain | Genotype | Vector | Reference |
|---|---|---|---|
| Δ*frmA::ka n* | BW25113 Δ*frmA*::kan | | Keio collection #JW0347 https://cgsc.biology.yale. edu/KeioList.php |
| Δ*tpiA:: kan* | BW25113 Δ*tpiA*::kan | | Keio collection #JW3890 https://cgsc.bioloqy.yale. edu/KeioList. php |
| Δ*frmAΔt pi A* | BW25113 Δ*frm* Δ*tpiA*::kan | pSEVA424 *mdh2* CT4-1 *Cupriavidus necator* pSEVA131 *hps phi Methylobacillus flagellatus* | This study, ancestral strain of the evolution experiment |

Long-term chemostat evolution

The evolution experiment was conducted in a 500 mL bioreactor (Multifors, Infors-HT, Bottmingen, Switzerland) filled with 300 mL minimal medium at 37°C under constant stirring (700 revolutions per minute (r.p.m.)) and aerated with compressed air. The bioreactor was equipped with medium feed, efflux, acid, and base pump systems. The pH was kept constant at 7.1 by the addition of either hydrochloric acid (HCl) or sodium hydroxide (NaOH). The efflux pump was operated at much higher speed than the feed pump to keep the volume (292, 296, and 299 mL for reactor 1, 2, and 3, respectively) of the culture constant and to maintain the chemostat condition. The culture volume and flow rate were determined by measuring the difference in weight of the feed and waste medium over time. The dilution rates were 0.42, 0.34, and 0.62 $d^{-1}$ for reactor 1, 2, and 3, respectively. The feed medium consisted of minimal medium supplemented with 500 mM methanol, 20 mM pyruvate, 0.1 mM IPTG, ampicillin, and streptomycin for the first 90 generations. Then, the pyruvate concentration was reduced to 10 mM and after another 6 generations to 5 mM, respectively. The state of the culture in the chemostat was followed by measuring the optical density of the medium at 600 nm. The chemostat was restarted twice, once after generations 202 generations (contamination) and once after 223 generations (fresh chemostat set at faster dilution rate). The doubling time of the bacterial culture and the number of generations were calculated from standard chemostat equations. At regular intervals, the population was tested for methylotrophic and methanol-dependent growth. To verify that the pyruvate concentration was limiting, sterile-filtered medium aliquots were analyzed by HPLC (UPLC Ultimate 3000, ThermoFisher Scientific, Reinach, Switzerland) equipped with an ion exclusion column (Rezex ROA-Organic Acid H+ (8%) 300 $\times$ 7.8mm, Phenomenex, Torrance, CA, United States of America) applying 5 mM $H_2SO_4$ as a mobile phase isocratically. 10 $\mu$L of sample were injected at a flow rate of 0.6 mL $min^{-1}$ and the absorbance at 210 nm was recorded for 25 minutes. The pyruvate concentration was calculated based on a standard curve from samples with known pyruvate levels (0.01, 0.1, 0.5, 1, 5, 10, 20 mM pyruvate) and concentrations below 0.01 mM were considered as below the detection limit.

Serial transfer evolution

After evolution in a chemostat for 249 generations, the population was propagated under a serial transfer regime. Initially, an aliquot from the chemostat population was passaged seven times in 20 mL minimal medium containing 500 mM methanol, 0.1 mM IPTG, Amp, and Sm. This culture was then used to inoculate four replicate lineages. Each was propagated in 30 mL of the same medium, except that IPTG was omitted, and passaged during mid- or late-exponential phase. All cultures were incubated in 100 mL baffled shake flasks at 37°C, 160 r.p.m. in a Minitron shaker (Infors-HT, Bottmingen, Switzerland). To inoculate fresh medium, old cultures were diluted 1:100 (V/V).

Characterization of the growth phenotype after 534 generations

After 534 generations of evolution, the four replicate lineages were streaked out on agar plates containing minimal medium supplemented with 500 mM methanol, ampicillin, and streptomycin. Four colonies were inoculated into 30 mL medium supplemented with 500 mM methanol, ampicillin, and streptomycin and cultivated in baffled shake flasks at 37°C, 160 r.p.m. in a Minitron shaker. During late-exponential growth, cultures were diluted 1:100 (V/V) in fresh medium of the same composition to assess growth. Growth of the bacterial cultures was monitored by measuring the $OD_{600}$ over time. A cryostock was generated of the fastest growing replicate (MEcoli_ref_1).

Proteome comparison between ancestral methanol-dependent *E. coli and* MEcoli ref 1.

MEcoli_ref_1 was streaked out on agar plate containing minimal medium supplemented with 500 mM methanol and incubated at 37°C until colonies were visible. A cross-section of colonies was used to inoculate a pre-culture in 30 mL minimal medium supplemented with 500 mM methanol and cultivated in baffled shake flasks at 37°C, 160 r.p.m. until stationary phase. Next, the culture was diluted 1:100 (V/V) into fresh medium, grown until mid-exponential phase and split 1:100 (V/V) into five main-culture replicates. Once the cultures reached mid-exponential phase ($OD_{600} \approx 0.6$), 4 OD units (1 OD unit equals 1 mL of culture at $OD_{600}$ of 1) of cells were harvested, cooled to 4°C, spun down (3220 g, 15 min), and washed once with 4 mL 10 mM $MgCl_2$, and twice with 1 mL 10 mM $MgCl_2$. Finally, the supernatant was discarded, the cell pellet shock frozen in liquid nitrogen and frozen. The same procedure was followed for the ancestral strain except all media were additionally supplemented with 20 mM pyruvate and 0.1 mM IPTG.

Cell pellets were dissolved in 300 $\mu$L 100 mM ammonium bicarbonate, 8 M urea, 1x cOmplete EDTA-free protease inhibitor cocktail (Sigma-Aldrich, Buchs, Switzerland) and lysed by indirect sonication (3 $\times$ 1 min, 100% amplitude, 0.8 s cycle time) in a VialTweeter (HIFU, Hielscher, Teltow, Germany). Larger particles and insoluble parts were removed by centrifugation at 13,000 g, 15 min, 4°C. Protein concentrations in the lysates were determined by Pierce BCA assays (Thermo Fischer Scientific, Reinach, Switzerland). Protein disulfide bonds were reduced by adding tris(2-carboxylethyl)phosphine (TCEP, Sigma-Aldrich, Buchs, Switzerland) to a final concentration of 5 mM and incubation for 30 min at 37°C and 300 r.p.m. shaking. Cysteine residues were alkylated by adding iodoacetamide (IAA, Sigma-Aldrich, Buchs, Switzerland) to a final concentration of 10 mM for 30 min at room temperature in the dark. Prior to digestion the urea concentration was reduced below 2 M by diluting all samples 1 to 5 with freshly prepared 50 mM ammonium bicarbonate. For digestion, sequencing grade modified trypsin (Promega AG, Dübendorf, Switzerland) was added at a 1:50 (μg trypsin/μg protein) ratio. Digestion was carried out in a tabletop shaker over night at 37°C under constant shaking at 300 r.p.m. After digestion, the trypsin was inactivated using heat incubation in a tabletop shaker at 95°C for 5 min and subsequent acidification to an approximate final concentration of 1% (V/V) formic acid. The peptide samples were centrifuged at 20,000 g for 10 min to remove insoluble parts and the supernatant was taken and desalted using Sep-Pak Vak C18 reversed phase columns (Waters Corporation, Baden-Dättswil, Switzerland) as described previously and dried under vacuum. Prior to MS analysis, the samples were re-solubilized in 3% acetonitrile (ACN) containing 0.1% formic acid (FA) to a final concentration of 0.5-1 μg μL$^{-1}$.

Mass spectrometry analyses was performed on an Orbitrap Lumos Tribrid mass spectrometer (Thermo Fischer Scientific) equipped with a digital PicoView source (New Objective, Littleton, USA) coupled to an M-Class ultraperformance liquid chromatography (UPLC) system (Waters GmbH, Wilmslow, UK). A two-channel solvent system was used with 0.1% formic acid (V/V) in water for channel A and 0.1% formic acid (V/V), 99.9% ACN (V/V) for channel B. At a peptide concentration of 0.5 μg μL$^{-1}$ for each sample 2 μL were loaded on an ACQUITY UPLC M-Class Symmetry C18 trap column (100 Å, 5 μm; 180 μm × 20 mm, Waters) followed by an ACQUITY UPLC M-Class HSS T3 column (100 Å, 1.8 μm; 75 μm × 250 mm, Waters). Peptide samples were separated at a flow rate of 300 nL min$^{-1}$ with an initial of 5% B for 3 min. The gradient was as follows: from 5% to 22% B in 112 min, from 22% to 32% B in 8 min, from 32% to 95% B in 5 min and from 95% to 5% B in 10 min. The mass spectrometer was operated in data-dependent acquisition (DDA) and full-scan mass spectra were acquired in the Orbitrap analyzer with a mass range of 300-2000 m/z and a resolution of 120k with an automated gain control (AGC) target value of 500,000. Fragment ion spectra (MS/MS) were acquired in the Ion trap using quadrupole isolation with a window of 1.6 Da and fragmented using higher energy collisional dissociation (HCD) with a normalized collision energy of 35%. Only precursor ions with charge states +2 to +7 and a signal intensity of at least 5000 were selected for fragmentation, and the maximum cycle time was set to 3 s. The ion trap was operated in rapid scan mode with an AGC target value of 10,000 and a maximum injection time of 50 ms. The dynamic exclusion was set to 25 s, and the exclusion window was set to 10 p.p.m. Measurements were acquired using internal lock mass calibration on m/z 371.10124 and 445.12003. Sample acquisition was performed in randomized order.

Progenesis QI (Nonlinear Dynamics, v.4.2.7207.22925) was used to process the acquired raw mass spectrometry data. Prior to the automatic alignment, 3-5 vectors were manually seeded to aid the alignment. As alignment reference a 1:1 pool of all samples was used. After normalization, from each peptide ion a maximum of the top five tandem mass spectra were exported. The mascot generic file (* .mgf) was searched using the Mascot server (Matrix Science, v.2.7.0.1) against a decoyed and reversed protein sequence database. Two databases were constructed: For the ancestral strain, one containing the 4449 annotated proteins of the ancestral strain BW25113 (Genbank accession: NZ_CP009273) supplemented with the amino acid sequences of Mdh, Hps, Phi and concatenated with the yeast proteome (Uniprot accession: UP000002311) as well as 260 known mass spectrometry contaminants. For the evolved strain, the same database, but with modified amino acid sequences to account for observed mutations. The Mascot search parameters were as follows: Precursor ion and fragment ion tolerance were set to ± 10 ppm and ± 0.5 Da, respectively. Trypsin was selected as protease (two missed cleavages) and ions with charge state 2+, 3+ am were 4+ were selected for identification. Carbamidomethylation of cysteine was set as fixed modification and oxidation of methionine, carbamylation of the N-terminus and lysine were set as variable modifications. The mascot search was imported into Scaffold (Proteome Software, v.5.1.0) using 5% peptide and 10% protein false discovery rate (FDR) and the resulting scaffold spectrum report were imported into Progenesis QI. For label-free protein quantification the Hi-3 approach was selected and only proteins with at least two unique peptides were considered for quantification. Statistical testing was performed directly in Progenesis with a one-way ANOVA and the resulting P-values were adjusted for multiple hypothesis testing using the Benjamini-Hochberg procedure (termed q-values). General cutoffs for significantly regulated proteins were q-values < 0.05 and | Log$_2$(fold-changes) | ≥ 1.5.

Relative proteome contributions of individual proteins were estimated as described previously in. Only proteins for which more than three peptides were detected were considered for analysis (i.e. the quantifiable proteome). Next, for each protein the mean peak area was divided by the mean sum of all peaks. This ratio determined the relative protein abundance of a protein.

Proteomics gene set enrichment analysis of KEGG pathways

Gene set enrichment analysis of the proteomics data was conducted against the KEGG database using clusterProfiler (gseKEGG) with the following settings: organism = 'eco', minGSSize = 3, pvalueCutoff = 0.05, pAdjustMethod = 'BH'.

Characterization of the growth phenotype of MEcoli ref 1

One of the replicates of the last proteomics preculture was diluted 1:100 (V/V) into minimal medium supplemented with 500 mM methanol. In the negative control methanol was omitted. The cultures were split into 10 technical replicates of 150 μL each, transferred to a 96 well microtiter plate and incubated at 37°C, 800 r.p.m. in a LogPhase 600 reader (Agilent, Basel, Switzerland). Growth was observed by measuring absorbance at 600 nm. A calibration curve was used to convert measured absorbance values to $OD_{600}$ values corresponding to measurements with a pathlength of 10 mm.

### $^{13}C$ isotopic tracer analysis of metabolites

After 368 generations of evolution, the four replicate serial dilution lineages were streaked out on agar plates containing minimal medium supplemented with 500 mM methanol, ampicillin, and streptomycin. Four colonies were inoculated into two different conditions: (1) 30 mL of minimal medium supplemented with 500 mM $^{13}C$ methanol (isotopic purity 99%, Euriso-Top GmbH, Saarbrücken, Germany), but without $Na_2EDTA$ and without antibiotics in baffled shake flasks, which were incubated at 37°C, 160 r.p.m. in a Minitron shaker under ambient atmosphere. (2) The same medium, which was sparged with synthetic air (80% (V/V) $N_2$, 20% (V/V) $O_2$) for 30 min before inoculation, in sealed shake flasks with a synthetic air atmosphere containing 5% (V/V) $^{13}CO_2$ (99% isotopic purity). In both conditions cultures were incubated at 37°C, 160 r.p.m in a Minitron shaker. At late-exponential phase cultures were diluted 1:100 (V/V) into identical conditions.

Once cultures reached optical densities between 0.5 and 1.21, metabolites were extracted from 7 to 10 OD units (1 OD unit equals 1 mL of culture at $OD_{600}$ of 1) of culture by rapid filtration. To this end, 5 OD units of culture were applied onto a 0.2 μm regenerated cellulose filter (RC58, Whatman GmbH, Dassel, Germany), filtered, washed with 10 mL, 37°C ultrapure water containing 500 mM $^{13}C$ methanol, quenched in 8 mL ice cold acetonitrile/methanol/0.5 M formic acid (60:20:20 (V/V/V)), vortexed for 10 s, and kept on ice for 10 min. Each culture was sampled twice in rapid succession. Following metabolite extraction, samples were lyophilized and subsequently resuspended in 250 μL solvent A/solvent B (90:10 (V/V), see below), centrifuged at 10,000 g, 4°C for 10 min, the supernatant transferred to a fresh tube and centrifuges again at 20,000 g, 4°C for 10 min and the supernatant transferred into HPLC vials. Assuming 1 OD unit corresponds to 250 μg cell dry weight, each sample was extracted from 2500 μg cell biomass dry weight, except samples from replicate 1 grown under ambient atmosphere and from replicate 4 grown under at 5% (V/V) $^{13}CO_2$, which were extracted from 2250 μg cell biomass dry weight and 1775 μg cell biomass dry weight, respectively, because lower culture volumes were sampled.

Metabolites were analyzed using ultra-high pressure liquid chromatography (UPLC Ultimate 3000, ThermoFisher Scientific, Reinach, Switzerland) equipped with a hydrophilic interaction liquid chromatography (HILIC) column (InfinityLab Poroshell 120 HILIC-Z; 2.1×100mm, 1.9um, Agilent Technologies, Basel, Switzerland) coupled to a hybrid quadrupole-orbitrap mass spectrometer (Q Exactive Plus, ThermoFisher Scientific, Reinach, Switzerland). The solvent system consisted of 10 mM ammonium acetate, 7 μM medronic acid in ultra-pure water, pH 9 (solvent A) and 10 mM ammonium acetate, 7 μM medronic acid in acetonitrile/ultra-pure water (90:10 (V/V)), pH = 9 (solvent B). To separate metabolites, the following gradient was used for elution at a constant flow rate of 500 μL / min: 10% A for 1 minute; linearly increased to 40% A over 5 minutes; 40% A for 3 min; linearly decreased to 10% A over 0.5 minutes; and held at 10% A for 3.5 minutes. As setting for the mass spectrometry part of the method, Fourier transform mass spectrometry in negative mode with a spray voltage of -2.8 kV, a capillary temperature of 275°C, S-lens RF level of 50, an auxiliary gas flow rate of 20, and an auxiliary gas heater temperature of 350°C was applied. Mass spectra were recorded as centroids at a resolution of 70'000 at mass to charge ratio (m/z) 200 with a mass range of 75 - 800 m/z and a scan rate of ~4 Hz in full scan mode was used. Of each sample 5 uL were injected.

LC-MS results were analyzed using the emzed framework (emzed.ethz.ch). Metabolite isotopologue peaks were extracted by a targeted approach using commercial standards to define retention time - m/z peak windows applying a m/z tolerance of ±0.002 Da. In case of the metabolite P5P, a mass tolerance of 0.0015 mass units was used for the analysis. The peak area cut off was set at 20,000 counts $s^{-1}$ $μL^{-1}$ injected. For the metabolite group 2-phosphoglycerate and 3-phosphoglycerate (2PG/3PG), only 3-phosphoglycerate was verified by a commercial standard. Isotopologue fractions ($s_i$) and labeled fraction (LF) were determined as previously described by targeted peak integration of all detected isotopologues utilizing Eq. (1) and Eq. (2) based on m, the abundance of the respective isotopologue; n, the number of carbons in the metabolite of interest; I and j, the isotopologues.

$$s_i = \frac{m_i}{\sum_{j=0}^{n} m_j} \quad (1)$$

$$LF = \frac{\sum_{i=0}^{n} m_i * i}{n * \sum_{i=0}^{n} m_i} \quad (2)$$

Probably due to technical issues, the extracted metabolome of colony 4 grown at enriched $^{13}CO_2$ atmosphere exhibited low to undetectable metabolite concentrations and was not considered for further analysis.

$^{13}$C isotopic tracer analysis of protein-bound amino acids and total biomass

Generation of samples for tracer analysis of protein-bound amino acids was identical to ones generated for metabolite analysis. Briefly, MEcoli_ref_1 was streaked out on agar plate containing minimal medium supplemented with 500 mM methanol and incubated at 37°C until colonies were visible. A cross-section of colonies was used to inoculate a preculture in 30 mL minimal medium supplemented with 500 mM methanol and cultivated in baffled shake flasks at 37°C, 160 r.p.m. until stationary phase. The preculture was split into three replicates of identical conditions as described above. Cells were harvested between $OD_{600}$ 0.5 and 1.8.

Protein-bound amino acids were isolated following previously established protocols: Cell pellets were resuspended in 200 μL 6 M HCl and baked at 105°C overnight. The cell hydrolysate was dried at 95°C under constant airflow, resuspended in 1 mL water and centrifuged twice at 20,000 g for 10 min to remove insoluble debris. Samples were diluted 1:100 (V/V) in starting conditions of the LC/MS method and analyzed as described above.

$^{13}$C labeling of total biomass samples was conducted by Imprint Analytics (Imprint Analytics GmbH, Neutal, Austria) by elemental analyzer/isotope ratio mass spectrometry.

Genome resequencing

For genome resequencing, about 2 OD units of cells were sampled, centrifuged for 1 minute at 11'000 g and the supernatant discarded. Genomic DNA was extracted by MasterPure DNA purification kit (Epicentre). Purified genomic DNA was sent for Illumina NovaSeq sequencing (Novogene UK, Cambridge United Kingdom). BBMap (38.95) clumpify function was used to filter raw reads for optical and PCR duplicates. The maximum distance to consider for optical replicates was set to the appropriate value for the used sequencer (dupedist = 12000) and we allowed for one base substitution between duplicates (subs = 1). For PCR duplicates, the same substitution setting was used with two passes for error correction (passes = 2). Filtered reads were aligned to the reference genome of *E. coli* BW25113 (Genbank accession: CP009273) and the plasmid maps of pSEVA424 *mdh2* CT4-1 *Cupriavidus necator* and pSEVA131 *hps phi Methylobacillus flagellatus* by Breseq (0.36.0) with default settings.

KEGG pathway enrichment of mutations

The set of mutations in MEcoli_ref_1 was analyzed for enrichment of KEGG pathway annotations using clusterProfiler (enrichKEGG) with the following settings: organism = 'eco', pvalueCutoff = 0.05, qvalueCutoff = 0.2, minGSSize = 3).

Methanol dehydrogenase activity assay

The ancestral and mutated DNA sequences encoding methanol dehydrogenase were cloned into a pET16b expression vector using Gibson assembly. The resulting constructs added ten histamine residues to the N-terminus of the enzymes for nickel-immobilized metal affinity chromatography purification.

For protein expression both constructs were cultured under the same conditions: A preculture was inoculated in 10 mL LB medium supplemented with carbenicillin and incubated at 37°C, 160 r.p.m. and diluted 1:50 (V/V) in 400 mL of the same medium in 2 L baffled shake flasks on the next day. The culture was grown to mid-exponential phase ($OD_{600}$ ~ 0.7) at 37°C, 160 r.p.m. Once an $OD_{600}$ of about 0.7 was reached, the culture was induced with 0.3 mM IPTG and incubated overnight at 16°C, 160 r.p.m.

Cells were harvested by centrifugation (3250 g, 30 min, 4°C), resuspended in 10 mL lysis buffer (50 mM $NaH_2PO_4$, 300 mM NaCl, 20 mM imidazole, 2 mM dithiothreitol, Roche cOmplete EDTA free protease inhibitor cocktail (Sigma-Aldrich Chemie GmbH, Buchs, Switzerland)) and lysed by sonication (6 mm sonication probe, amplitude 30, process time 4 min, impulse time 5 s, cool down time 15 s, Q700 sonicator (Qsonica LLC, U.S.A.)). Cell debris was cleared by centrifugation (20,000 g, 40 min, 4°C). Methanol dehydrogenase was isolated from the resulting solution by fast protein liquid chromatography (ÄKTA, HisTrap HP, GE Healthcare, Chicago, U.S.A.) using a linear gradient from starting buffer (lysis buffer without protease inhibitor cocktail) to elution buffer (starting buffer with 500 mM imidazole) over 20 min at a flow rate of 1 mL min$^{-1}$. Buffer was exchanged to reaction buffer (100 mM MOPS, 5 mM $MgSO_4$) by repeated concentration in centrifugal filter units (Amicon Ultra, 10 kDA molecular mass cutoff, Sigma-Aldrich Chemie GmbH, Buchs, Switzerland) and subsequent dilution in reaction buffer until a total dilution factor of greater than 100,000 was achieved.

Methanol dehydrogenase activity was assayed in reaction buffer supplemented with 5 mM nicotinamide adenine dinucleotide (NAD$^+$) as well as 500 mM methanol at 37°C and by following the formation for NADH/H$^+$, i.e. measuring absorbance at 380 nm in a microplate reader (Tecan Infinite Pro 200, Tecan Group Ltd., Männedorf, Switzerland). Both methanol dehydrogenase variants were added to the reaction mix at equal concentration.

**Claims**

1. A recombinant non-naturally occurring methylotrophic microorganism,

   - which expresses or over-expresses a polypeptide having methanol dehydrogenase activity, a polypeptide having 3-hexulose-6-phosphate activity, and polypeptide having a 6-phospho 3-hexuloisomerase activity, and
   - which does not comprise, and/or comprises deletions or reductions of expression of, or eliminations or reductions of activity of a polypeptide having triose-phosphate-isomerase activity and of a polypeptide having glutathione-dependent formaldehyde dehydrogenase activity,

   wherein the recombinant non-naturally occurring methylotrophic microorganism grows on one carbon (C1) compounds as sole carbon source, with at least 50% of the carbon source consisting of one or more reduced C1 carbon compounds.

2. The recombinant methylotrophic microorganism of claim 1, which, when grown on C1 compounds, uses at least 70%, at least 75%, at least 80%, or 90% $\pm$ 5% of reduced C1 compounds, preferably methanol, and the remainder of another C1 compound, such as carbon dioxide, as its sole carbon source.

3. The recombinant methylotrophic microorganism of claims 1 or 2, expressing or over expressing all enzymes required for a complete ribulose monophosphate (RuMP) cycle, consisting of a polypeptide having methanol dehydrogenase activity, such as Mdh, a polypeptide having 3-hexulose 6-phosphate synthase, such as Hps, a polypeptide having 6-phospho 3-hexuloisomerase activity, such as Phi, a polypeptide having glucose-6-phosphate isomerase activity, such as Pgi, a polypeptide having transketolase activity, such as TktA, a polypeptide having ribulose-phosphate 3-epimerase, such as Rpe, a polypeptide having ribose-5-phosphate isomerase activity, such as RpiA, a polypeptide having transaldolase activity, such as TalA, apolypeptide having NADP$^+$-dependent glucose-6-phosphate dehydrogenase, such as Zwf, a polypeptide having 2-keto-3-deoxygluconate 6-phosphate/2-keto-4-hydroxyglutarate aldolase activity, such as Eda, and a polypeptide having a phosphogluconate dehydratase, such as Edd.

4. The recombinant methylotrophic microorganism of any of claims 1 to 3, expressing or over-expressing a plurality of enzymes selected from the group consisting of of Mdh, Hps, Phi, TktA, TktB, Rpe, RpiA, RpiB, TalA, TalB, Edd, and Eda,, or any combination thereof.

5. The synthetic methylotrophic microorganism of any of claims 1 to 4, expressing or over-expressing a polypeptide having

   - TktA activity and a polypeptide having RpiB activity, or
   - a polypeptide having TktA activity and a polypeptide having Edd or Eda activity, or
   - a polypeptide having RpiB and a polypeptide having Edd or Eda activity.

6. The recombinant methylotrophic microorganism of any of claims 1 to 5, expressing or over-expressing a truncated GntR polypeptide, in particular a truncation in the effector binding /oligomerization domain, or an at least partial deletion of the effector binding domain and/or oligomerization domain of the GntR polypeptide, for example a truncation at residue 312 of the GntR polypeptide.

7. The recombinant methylotrophic microorganism of any of claims 1 to 6, comprising a reduction in expression, or a deletion of a native gene selected from the group consisting of *gntR, tktB, pgi, pkg, pgl, gnd, aroH, aroF, pfkA, prs, purF* a gene encoding an enzyme of the tricarboxylic acid (TCA) cycle, including *aceA, acnA, fumC, sdhB, sucA, sucC, icd, aceE, aceF, sdhA, sucC, and mqo,* or any combination thereof.

8. The recombinant methylotrophic microorganism of any of claims 1 to 7, comprising a reduction in expression, or a deletion of the gene encoding the Gnd polypeptide and in the gene encoding the AroH polypeptide.

9. The recombinant methylotrophic microorganism of any of claims 1 to 8, expressing or over-expressing a polypeptide having a methanol dehydrogenase activity and having at least 70%, 80%, 90%, 95%, 98% or 99% sequence identity to SEQ ID NO: 1, wherein the polypeptide comprises at least an H165N mutation.

10. The recombinant methylotrophic microorganism of any of claims 1 to 9, capable of reaching an optical density at 600 nm ($OD_{600}$) of 2 or more, or of 2.5 or more when grown on methanol.

11. The recombinant methylotrophic microorganism of any of claims 1 to 10, capable of growing on methanol at a doubling time of 10 hours or less, preferably of 8 hours or less.

12. The recombinant methylotrophic microorganism of any of claims 1 to 11, wherein the microorganism is obtained by genetically modifying a non-naturally occuring methylotrophic microorganism selected from a group consisting of *Escherichia*, in particular *Escherichia coli, Bacillus*, in particular *Bacillus subtilis, Clostridium, Enterobacter, Klebsiella, Mannheimia, Pseudomonas*, in particular, *Pseudomonas putida, Acinetobacter, Shewanella, Paracoccus, Ralstonia, Geobacter, Zymomonas, Acetobacter, Geobacillus, Lactococcus, Streptococcus, Lactobacillus, Corynebacterium*, in particular *Corynebacterium glutamicum, Streptomyces, Proprionibacterium, Synechocystis, Synechococcus, Cyanobacteria, Chlorobi, Deinococcus* and *Saccharomyces sp.*

13. The recombinant methylotrophic microorganism of any of claims 1 to 12, which is the designated *Escherichia coli* MEcoli_ref_1 having CCOS accession number CCOS 2032, or an *Escherichia coli* strain having a doubling time and product profile of CCOS deposit accession number CCOS 2032.

14. A Method for producing or for increasing production of a metabolite, comprising the steps of growing the recombinant microorganism of any of claims 1 to 13 on a C1 compound and obtaining the metabolite.

15. The method of claim 14, wherein the metabolite is selected from a group consisting of formate, 2-carbon compounds, 3-carbon compounds, such as lactate, 4-carbon compounds, higher carboxylic acids, alcohols of higher carboxylic acids, polyhydroxyalkanoates, speciality chemicals, aromatic compounds, vitamins and their derivatives or precursors, carotenoids, amino acids, or sugars, such as deoxyribose.

Fig. 1A

Fig. 1B

Fig. 2

Fig. 3

Fig. 4A

Fig. 4B

**Fig. 5A**

EP 4 310 174 A1

Fig. 5B

Fig. 6A

Fig. 6B

$^{13}C$ methanol + $^{13}CO_2$

isotopologue fraction

number of labeled carbons

n
n-1
n-2
n-3
n-4
n-5

Fig. 6C

C methanol + $^{13}CO_2$

$^{13}C$ labeled fraction

Fig. 6D

Fig. 7A

Fig. 7B

Fig. 7C

Fig. 7D

Fig. 8

Fig. 9

Europäisches
Patentamt

European
Patent Office

Office européen
des brevets

# EUROPEAN SEARCH REPORT

Application Number

EP 22 18 5402

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2022/015796 A1 (ACADEMIA SINICA; UNIV CALIFORNIA [US]) 20 January 2022 (2022-01-20) * claim; figure; example * | 1-15 | INV. C12N1/00 |
| A | WO 2020/006058 A2 (GENOMATICA INC [US]) 2 January 2020 (2020-01-02) * claim; figure; example * | 1-15 | |
| A,D | WO 2018/148703 A1 (PAPOUTSAKIS ELEFTHERIOS T [US]; BENNETT ROBERT KYLE [US] ET AL.) 16 August 2018 (2018-08-16) * claim; figure; example * | 1-15 | |
| A,D | KELLER PHILIPP ET AL: "Methanol-dependent Escherichia coli strains with a complete ribulose monophosphate cycle", NATURE COMMUNICATIONS, vol. 11, no. 1, 26 October 2020 (2020-10-26), XP93021033, DOI: 10.1038/s41467-020-19235-5 Retrieved from the Internet: URL:https://www.nature.com/articles/s41467-020-19235-5.pdf> * the whole document * | 1-15 | |
| X,P | KELLER PHILIPP ET AL: "Generation of an Escherichia coli strain growing on methanol via the ribulose monophosphate cycle", NATURE COMMUNICATIONS, vol. 13, no. 1, 1 December 2022 (2022-12-01), XP93021048, DOI: 10.1038/s41467-022-32744-9 * the whole document * | 1-15 | |

TECHNICAL FIELDS
SEARCHED (IPC)

C12N
C12R

The present search report has been drawn up for all claims

| Place of search | Date of completion of the search | Examiner |
|---|---|---|
| Munich | 7 February 2023 | Luo, Xinmei |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 18 5402

This annex lists the patent family members relating to the patent documents cited in the above-mentioned European search report.
The members are as contained in the European Patent Office EDP file on
The European Patent Office is in no way liable for these particulars which are merely given for the purpose of information.

07-02-2023

| Patent document cited in search report | | Publication date | Patent family member(s) | | Publication date |
|---|---|---|---|---|---|
| WO 2022015796 | A1 | 20-01-2022 | TW | 202229541 A | 01-08-2022 |
| | | | WO | 2022015796 A1 | 20-01-2022 |
| WO 2020006058 | A2 | 02-01-2020 | CA | 3103377 A1 | 02-01-2020 |
| | | | CN | 113166784 A | 23-07-2021 |
| | | | EP | 3814515 A2 | 05-05-2021 |
| | | | JP | 2021528977 A | 28-10-2021 |
| | | | US | 2022177895 A1 | 09-06-2022 |
| | | | WO | 2020006058 A2 | 02-01-2020 |
| WO 2018148703 | A1 | 16-08-2018 | US | 2020017888 A1 | 16-01-2020 |
| | | | WO | 2018148703 A1 | 16-08-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018148703 A

- WO 2022015796 A

**Non-patent literature cited in the description**

- **KIM S. et al.** *Nat. Chem. Biol.,* 2020
- **KELLER, P. et al.** *Nat. Commun.,* 2020, vol. 11, 5403, https://doi.org/10.1038/s41467-020-19235-5
- **WU, T.-Y. et al.** *Appl. Microbiol. Biotechnol.,* 2016, vol. 100, 4969-4983
- **KELLER P. et al.** Methanol-dependent Escherichia coli strains with a complete ribulose monophosphate cycle. *Nat. Commun.,* 2020, vol. 11, 1-10
- **MEYER, F. et al.** Methanol-essential growth of Escherichia coli. *Nat. Commun.,* 2018, vol. 9